# EUROPEAN PATENT SPECIFICATION

(11) **EP 3 173 482 B1**
(45) Date of publication and mention of the grant of the patent: **01.01.2020**
(21) Application number: 15818679.1
(22) Date of filing: 08.07.2015
(51) Int. Cl.: C07K 16/32

(54) **RNA-PROTEIN COMPLEX, AND RNA AND PROTEIN DELIVERY SYSTEM USING SAME**
RNA-PROTEIN-KOMPLEX UND RNA- UND PROTEINABGABESYSTEM DAMIT
COMPLEXE ARN-PROTÉINE, ET SYSTÈME D'ADMINISTRATION D'ARN ET DE PROTÉINE À L'AIDE DUDIT COMPLEXE

(30) Priority: 09.07.2014 JP 2014141431
(43) Date of publication of application: 31.05.2017
(73) Proprietor: Kyoto University, Kyoto-shi, Kyoto 606-8501 (JP)
(72) Inventor: SAITO, Hirohide, Kyoto-shi Kyoto 606-8501 (JP); OSADA, Eriko, Kyoto-shi Kyoto 606-8501 (JP)
(74) Representative: HGF Limited
(86) International application number: PCT/JP2015/069634
(87) International publication number: WO 2016/006628

(56) References cited:
- WO-A1-2005/079843
- WO-A1-2010/010862
- WO-A1-2010/067811
- JP-A- 2007 537 700
- US-A1- 2011 263 026
- STEPHANIE A. KAZANE ET AL: "Self-Assembled Antibody Multimers through Peptide Nucleic Acid Conjugation", JOURNAL OF THE AMERICAN CHEMICAL SOCIETY, vol. 135, no. 1, 9 January 2013 (2013-01-09), pages 340-346, XP055076261, ISSN: 0002-7863, DOI: 10.1021/ja309505c
- HIROHISA OHNO ET AL: "Synthetic RNA-protein complex shaped like an equilateral triangle", NATURE NANOTECHNOLOGY, vol. 6, no. 2, 1 February 2011 (2011-02-01), pages 116-120, XP055346447, GB ISSN: 1748-3387, DOI: 10.1038/nnano.2010.268
- ERIKO OSADA ET AL: "Engineering RNA-Protein Complexes with Different Shapes for Imaging and Therapeutic Applications", ACS NANO, vol. 8, no. 8, 26 August 2014 (2014-08-26) , pages 8130-8140, XP055346446, US ISSN: 1936-0851, DOI: 10.1021/nn502253c
- FUJITA YOSHIHIKO ET AL: "Cell-surface receptor control that depends on the size of a synthetic equilateral-triangular RNA-protein complex", SCIENTIFIC REPORTS, vol. 4, September 2014 (2014-09), XP002776783,
- OHNO, H. ET AL.: 'Synthetic RNA-protein complexshaped like an equilateral triangle.' NAT. NANOTECHNOL. vol. 6, 2011, pages 116 - 120, XP055346447
- HIROHIDE SAITO: 'RNA-Protein nanotechnology & synthetic biology' JOURNAL OF JAPANESE BIOCHEMICAL SOCIETY vol. 86, no. L, 25 February 2014, pages 81 - 85, XP008185526

## Description

### [Technical Field]

The present invention relates to an RNA-protein complex having a specific structure and to a method of using the same.

### [Background Art]

A nanoscale structure has been constructed using biomolecules in the field of nanotechnology. In addition, in the field of protein engineering, a novel creation of enzymes by molecular design has been attempted. The "material" used in such attempts is a nucleic acid or a protein.

As a structure produced using such a nucleic acid and a protein, a triangular structure has been reported (Patent Literature 1 and Non Patent Literature 1).

However, the function of a complex consisting of a nucleic acid and a protein having such a triangular structure was unknown, and there have been no specific reports regarding a method of using the same.

### [Citation List]

### [Patent Literature]

[Patent Literature 1] WO2010/010862

### [Non Patent Literature]

[Non Patent Literature 1] Ohno H, et al., Nat Nanotechnol. 2011, 6: 116-120.

### [Summary of Invention]

### [Technical Problem]

It is an object of the present disclosure to provide an RNA or protein delivery system utilizing the properties of an RNA-protein complex, or a functional RNA-protein complex having a novel structure.

### [Solution to Problem]

The present disclosure has been made to achieve the aforementioned object. That is to say, according to one instance the present disclosure is characterized in that it uses an RNA-protein complex comprising a protein specifically recognizing a cell surface antigen, and in that it uses the RNA-protein complex, while appropriately selecting the RNA structure, in order to further enhance the ability of the complex to recognize cells.

According to another instance the present disclosure (and the present invention) is characterized in that it utilizes an RNA-protein complex comprising functional RNA and a functional protein and allows the RNA-protein complex to exhibit such functions.

Described herein is:
[1] An RNA and protein delivery system using an RNA-protein complex comprising the following (1) to (3):
   (1) an RNA comprising the following sequences (i) to (iv):
      (i) three or more sequences each forming an RNA-protein binding motif,
      (ii) two or more sequences each consisting of 15 or more nucleotides,
      (iii) a 5'-terminal sequence, and
      (iv) a 3'-terminal sequence, wherein
         (iii) the 5'-terminal sequence and (iv) the 3'-terminal sequence have a total length of 15 or more nucleotides;
   (2) an RNA comprising the following sequences:
      (a) three or more sequences each forming an RNA-protein binding motif, together with (1) (i),
      (b) one or more sequences complementary to the sequences each consisting of 15 or more nucleotides described in (1) (ii),
      (c) sequences continuously complementary to (1) (iii) the 5'-terminal sequence and the 3'-terminal sequence,
      (d) a 5'-terminal sequence, and
      (e) a 3'-terminal sequence, wherein
         (d) the 5'-terminal sequence and (e) the 3'-terminal sequence are continuously complementary to the sequences each consisting of 15 or more nucleotides described in (1) (ii); and
   (3) at least one fusion protein comprising a protein forming an RNA-protein binding motif with the RNAs that are formed by (1) (i) and (2) (a), and a protein specifically recognizing a cell surface antigen.
[2] The system according to [1], wherein there are the three sequences each forming an RNA-protein binding motif described in (1) (i), there are the three sequences each forming an RNA-protein binding motif described in (2) (a), there are the two sequences each consisting of 15 or more nucleotides described in (1) (ii), and there is the one sequence complementary to the sequence consisting of 15 or more nucleotides described in (2) (b).
[3] The system according to [1] or [2], wherein the length of the sequence in (1), the total length of (1) (iii) the 5'-terminal sequence and (iv) the 3'-terminal sequence, the length of the sequence in (2) (b), or the total length of (2) (d) the 5'-terminal sequence and (e) the 3'-terminal sequence is 15 or more nucleotides and 92 or fewer nucleotides.
[4] The system according to any one of [1] to [3], wherein the length of the sequence in (1), the total length of (1) (iii) the 5'-terminal sequence and (iv) the 3'-terminal sequence, the length of the sequence in (2) (b), or the total length of (2) (d) the 5'-terminal sequence and (e) the 3'-terminal sequence is selected, as appropriate, so that the ability to bind to a cell surface antigen can be enhanced.
[5] The system according to any one of [1] to [4], wherein the RNA-protein binding motifs formed by (1) (i) and (2) (a) are K-turn motifs, and the protein forming an RNA-protein binding motif described in (3) is L7Ae.
[6] The system according to any one of [1] to [5], wherein the protein specifically recognizing a cell surface antigen described in (3) is a protein recognizing HER2.
[7] The system according to [6], wherein the protein recognizing HER2 is Affibody.
[8] The system according to any one of [1] to [7], wherein the delivery is carried out in the presence of serum.
   The present invention provides:
[9] An RNA-protein complex comprising the following (1) to (4):
   (1) an RNA comprising the following sequences (i) to (iii):
      (i) a sequence forming an RNA-protein binding motif,
      (ii) two sequences each consisting of seven or more nucleotides, and
      (iii) a siRNA sequence;
   (2) an RNA comprising the following sequences (a) to (d):
      (a) a sequence forming an RNA-protein binding motif, together with (1) (i),
      (b) two or more sequences each consisting of 14 or more nucleotides, which are continuously complementary to the two sequences each consisting of seven or more nucleotides described in (1) (ii),
      (c) a 5'-terminal sequence, and
      (d) a 3'-terminal sequence, wherein
         (c) the 5'-terminal sequence and (d) the 3'-terminal sequence are continuously complementary to the sequences each consisting of seven or more nucleotides described in (1) (ii);
   (3) at least one protein forming an RNA-protein binding motif with the RNAs formed by (1) (i) and (2) (a); and
   (4) an RNA comprising a sequence complementary to the siRNA sequence described in (1) (iii).
[10] The RNA-protein complex according to [9], wherein there are the three RNAs described in (1), there are the two sequences each consisting of 14 or more nucleotides described in (2) (b), and there are the three RNAs described in (4).
[11] The RNA-protein complex according to [9] or [10], wherein the sequence described in (1) (ii) consists of 7 or more nucleotides and 85 or fewer nucleotides, and the sequence described in (2) (b) consists of 14 or more nucleotides and 92 or fewer nucleotides.
[12] The RNA-protein complex according to any one of [9] to [11], wherein the RNA-protein binding motifs formed by (1) (i) and (2) (a) are K-turn motifs, and the protein described in (3) is L7Ae.
[13] The RNA-protein complex according to any one of [9] to [12], wherein the protein described in (3) is a fusion protein further comprising a protein specifically recognizing a cell surface antigen or a membrane-permeable peptide.
[14] The RNA-protein complex according to [13], wherein the membrane-permeable peptide is a peptide selected from the group consisting of AntP, HIV-derived TAT, Penetratin, Buforin II, Transportan, MAP, K-FGF, Ku70, Prion, pVEC, Pep-1, Pep-7, SynB1, HN-I, and HSV-derived VP22.

Also described herein is an RNA and protein delivery system using the RNA-protein complex according to any one of [9] to [14], and an RNA inhibitor comprising the RNA-protein complex according to any one of [9] to [14].

The invention further provides [15] a method for decomposing mRNA in cells, which comprises a step of contacting the RNA-protein complex according to any one of [9] to [14] with cells.

In the method, the RNA-protein complex may be contacted with the cells in the presence of serum.

Also described herein is a method for selecting the nucleotide length of each of the sequences in the following (1) (ii), the sequences in the following (1) (iii), the sequence(s) in the following (2) (b), and the sequences in the following (2) (d), using the ability to bind to a cell surface antigen as an indicator, in an RNA-protein complex comprising the following (1) to (3):
(1) an RNA comprising the following sequences:
   (i) three or more sequences each forming an RNA-protein binding motif,
   (ii) two or more sequences each consisting of 15 or more nucleotides, and
   (iii) a 5'-terminal sequence and a 3'-terminal sequence, which consist of a total of 15 or more nucleotides;
(2) an RNA comprising the following sequences:
   (a) three or more sequences each forming an RNA-protein binding motif, together with (1) (i),
   (b) one or more sequences complementary to the sequences each consisting of 15 or more nucleotides described in (1) (ii),
   (c) sequences continuously complementary to (1) (iii) the 5'-terminal sequence and the 3'-terminal sequence, and
   (d) a 5'-terminal sequence and a 3'-terminal sequence, which are continuously complementary to the sequences each consisting of 15 or more nucleotides described in (1) (ii); and
(3) at least one fusion protein comprising a protein forming an RNA-protein binding motif with the RNAs formed by (1) (i) and (2) (a) and a protein specifically recognizing a cell surface antigen.

The invention further provides [16] a method for selecting the nucleotide length of each of the sequences in the following (1) (ii) and the sequence(s) in the following (2) (b), using the ability to bind to a cell surface antigen as an indicator, in an RNA-protein complex comprising the following (1) to (4):
(1) an RNA comprising the following sequences (i) to (iii):
   (i) a sequence forming an RNA-protein binding motif,
   (ii) two sequences each consisting of seven or more nucleotides, and
   (iii) a siRNA sequence;
(2) an RNA comprising the following sequences (a) to (d):
   (a) a sequence forming an RNA-protein binding motif, together with (1) (i),
   (b) two or more sequences each consisting of 14 or more nucleotides, which are continuously complementary to the two sequences each consisting of seven or more nucleotides described in (1) (ii),
   (c) a 5'-terminal sequence, and
   (d) a 3'-terminal sequence, wherein
      (c) the 5'-terminal sequence and (d) the 3'-terminal sequence are continuously complementary to the sequences each consisting of seven or more nucleotides described in (1) (ii);
(3) at least one fusion protein comprising a protein forming an RNA-protein binding motif with the RNAs formed by (1) (i) and (2) (a) and a protein specifically recognizing a cell surface antigen; and
(4) an RNA comprising a sequence complementary to the siRNA sequence described in (1) (iii).

### [Advantageous Effects]

According to the present disclosure, by using the RNA-protein complex comprising a protein having the ability to bind to a cell surface antigen provided by the present disclosure, target cells can be recognized more strongly than in the case of the recognition ability that can be achieved by a single protein, and thus, it is advantageous. Moreover, even when an RNA-protein complex comprising functional RNA is used, the function of the RNA is exhibited. Accordingly, by using a combination of each functional protein and each functional RNA, an RNA-protein complex retaining desired functions can be provided, and thus, it is advantageous.

### [Brief Description of Drawings]

[Figure 1] Figure 1 shows the structure of RNA in each RNA-protein complex (LS-15 RNA, LS-26 RNA, LS-48 RNA, LS-70 RNA, and LS-92 RNA).

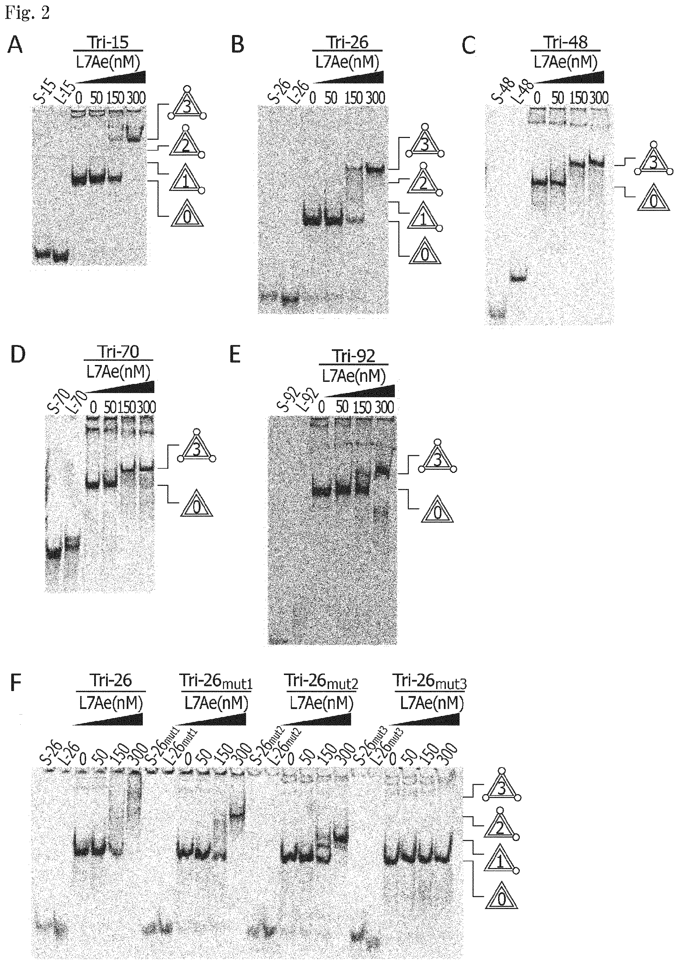

[Figure 2] Figure 2A shows the results of a gel shift assay, when L7Ae was added in different concentrations to LS-15 RNA. On the right side of the view, the structure of the assumed RNA-protein complex in each band is shown. Figure 2B shows the results of a gel shift assay, when L7Ae was added in different concentrations to LS-26 RNA. On the right side of the view, the structure of the assumed RNA-protein complex in each band is shown. Figure 2C shows the results of a gel shift assay, when L7Ae was added in different concentrations to LS-48 RNA. On the right side of the view, the structure of the assumed RNA-protein complex in each band is shown. Figure 2D shows the results of a gel shift assay, when L7Ae was added in different concentrations to LS-70 RNA. On the right side of the view, the structure of the assumed RNA-protein complex in each band is shown. Figure 2E shows the results of a gel shift assay, when L7Ae was added in different concentrations to LS-92 RNA. On the right side of the view, the structure of the assumed RNA-protein complex in each band is shown. Figure 2F shows the results of a gel shift assay, when L7Ae was added in different concentrations to LS-26, LS-26ₘᵤₜ₁, LS-26ₘᵤₜ₂, and LS-26ₘᵤₜ₃. On the right side of the view, the structure of the assumed RNA-protein complex in each band is shown.
[Figure 3] Figure 3 shows high speed AFM (HS-AFM) images, in a case in which L7Ae was added (+L7Ae), or was not added (-L7Ae) to RNA in each RNA-protein complex (LS-15 RNA, LS-26 RNA, LS-48 RNA, LS-70 RNA, and LS-92 RNA). Each upper view shows an enlarged view of the lower view.
[Figure 4] Figure 4 shows high speed AFM (HS-AFM) images, in a case in which L7Ae was added (+ L7Ae), or was not added (- L7Ae) to LS-26, LS-26ₘᵤₜ₁, LS-26ₘᵤₜ₂, and LS-26ₘᵤₜ₃.
[Figure 5] Figure 5 shows the results obtained by measuring the amount of L7Ae incorporated into each RNA-protein complex, in a case in which the L7Ae was added to LS-26, LS-26ₘᵤₜ₁, LS-26ₘᵤₜ₂, and LS-26ₘᵤₜ₃.
[Figure 6] Figure 6 shows time-lapse images (30 sec, 35 sec, and 85 sec) obtained by analyzing L7Ae-added LS-26 by high speed AFM (HS-AFM). The upper view shows a schematic view of the structure.
[Figure 7] Figure 7 shows the results of a gel shift assay obtained after LS-26 or an RNA-protein complex (Tri-26) was subjected to a Phenol treatment, addition of 20% FBS thereto, and incubation for 30 minutes, 60 minutes or 120 minutes.
[Figure 8] Figure 8 shows the results of a gel shift assay obtained after LS-26 or an RNA-protein complex (Tri-26) was subjected to a phenol treatment, addition of 20% human serum thereto, and incubation for 30 minutes, 60 minutes or 120 minutes.
[Figure 9] Figure 9A shows a schematic view of an RNA-protein complex having Affibody. Figure 9B shows the high speed AFM (HS-AFM) images of LS-26, to which an L7Ae-Affibody fusion was added (+L7Ae-Affibody), or was not added (-L7Ae-Affibody). The left view shows an enlarged view of the right view.

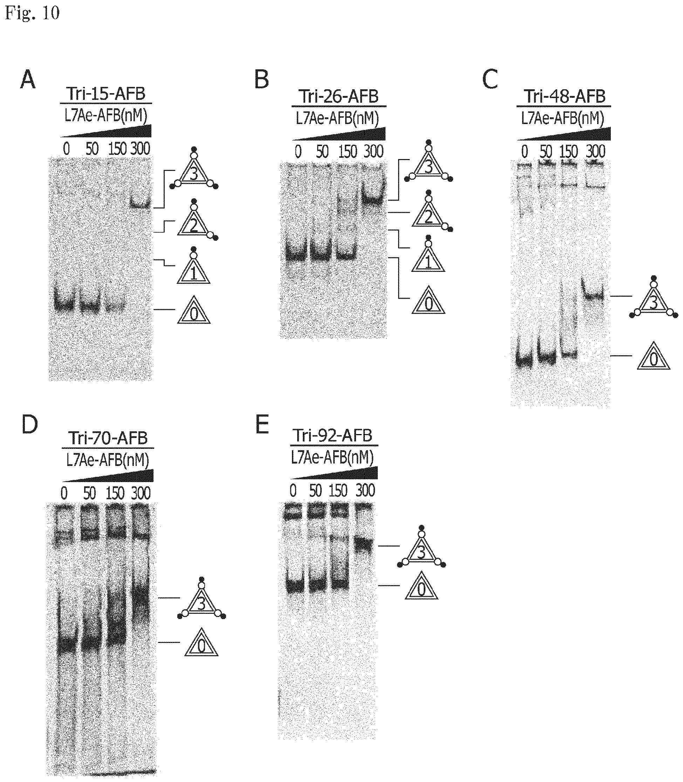

[Figure 10] Figure 10A shows the results of a gel shift assay, when an L7Ae-Affibody fusion was added in different concentrations to LS-15 RNA. On the right side of the view, the structure of the assumed RNA-protein complex in each band is shown. Figure 10B shows the results of a gel shift assay, when an L7Ae-Affibody fusion was added in different concentrations to LS-26 RNA. On the right side of the view, the structure of the assumed RNA-protein complex in each band is shown. Figure 10C shows the results of a gel shift assay, when an L7Ae-Affibody fusion was added in different concentrations to LS-48 RNA. On the right side of the view, the structure of the assumed RNA-protein complex in each band is shown. Figure 10D shows the results of a gel shift assay, when an L7Ae-Affibody fusion was added in different concentrations to LS-70 RNA. On the right side of the view, the structure of the assumed RNA-protein complex in each band is shown. Figure 10E shows the results of a gel shift assay, when an L7Ae-Affibody fusion was added in different concentrations to LS-92 RNA. On the right side of the view, the structure of the assumed RNA-protein complex in each band is shown.
[Figure 11] Figure 11 shows fluorescence intensity indicating the amount of an RNA-protein complex binding to cells of breast cancer cell line (SKBR3), when an RNA-protein complex having each Affibody (Tri-15-AFB, Tri-26-AFB, Tri-48-AFB, Tri-70-AFB, and Tri-92-AFB) was added to the breast cancer cell line.
[Figure 12] Figure 12A shows fluorescence intensity indicating the amount of an RNA-protein complex binding to cells of each breast cancer cell line (SKBR3, MCF-7 and MDA-MB-231), when the RNA-protein complex having Affibody (Tri-26-AFB) was added to the breast cancer cell line. Figure 12B shows stained images obtained after addition of the RNA-protein complex having Affibody (Tri-26-AFB) to each breast cancer cell line (SKBR3, MCF-7 and MDA-MB-231) (Alexa 647 (which stains RNA), and Hoechst (which stains cell nucleus)).
[Figure 13] Figure 13 shows fluorescence intensity indicating the amount of an RNA-protein complex binding to cells of breast cancer cell line (SKBR3), when an RNA-protein complex having each mutant motif and Affibody (Tri-26-AFB, Tri-26ₘᵤₜ₁-AFB, Tri-26ₘᵤₜ₂-AFB, and Tri-26ₘᵤₜ₃-AFB) was added to the breast cancer cell line.
[Figure 14] Figure 14 shows the structure of RNA in an RNA-protein complex (LS-26-siGFP RNA).
[Figure 15] Figure 15 shows the high speed AFM (HS-AFM) images of LS-26-siGFP RNA, to which L7Ae was added (+L7Ae), or was not added (-L7Ae). The left view shows an enlarged view of the right view.
[Figure 16] Figure 16 shows the results of a gel shift assay, in a case in which L7Ae was added or was not added to an RNA comprising different combinations of L1, L2, L3, S and AS, which are constituents of LS-26-siGFP RNA, or in a case in which sh-GFP used as a positive control was contacted with sh-GFP as a substrate in the presence or absence of Dicer.

### [Description of Instances]

Hereinafter, the present disclosure will be described in detail in the following instances. However, the following instances are not intended to limit the scope of the present disclosure.

According to one instance of the present disclosure, an RNA and protein delivery system using an RNA-protein complex is provided. The delivery system is a system for delivering a desired substance to a target, and in the present disclosure, such a delivery system can also be referred to as an RNA-protein complex for delivering RNA and/or a protein to cells.

One instance of the RNA-protein complex of the present disclosure is a first RNA-protein complex comprising the following (1) to (3):
(1) a first RNA comprising the following sequences (i) to (iv):
   (i) three or more sequences each forming an RNA-protein binding motif,
   (ii) two or more sequences each consisting of 15 or more nucleotides,
   (iii) a 5'-terminal sequence, and
   (iv) a 3'-terminal sequence, wherein
      (iii) the 5'-terminal sequence and (iv) the 3'-terminal sequence have a total length of 15 or more nucleotides;
(2) a second RNA comprising the following sequences (a) to (e):
   (a) three or more sequences each forming an RNA-protein binding motif, together with (1) (i),
   (b) one or more sequences complementary to the sequences each consisting of 15 or more nucleotides described in (1) (ii),
   (c) sequences continuously complementary to (1) (iii) the 5'-terminal sequence and the 3'-terminal sequence,
   (d) a 5'-terminal sequence, and
   (e) a 3'-terminal sequence, wherein
      (d) the 5'-terminal sequence and (e) the 3'-terminal sequence are continuously complementary to the sequences each consisting of 15 or more nucleotides described in (1) (ii); and
(3) at least one fusion protein comprising a protein forming an RNA-protein binding motif with the RNAs that are formed by (1) (i) and (2) (a), and a protein specifically recognizing a cell surface antigen.

In the present disclosure, (1) the RNA comprising (i) the sequence forming an RNA-protein binding motif means the side of RNA contained in an RNA-protein binding motif in a natural or known RNA-protein complex, or the side of RNA contained in an artificial RNA-protein binding motif obtained by an *in vitro* evolution method (*in vitro* selection method).

It has been known that a sequence forming a natural RNA-protein binding motif is generally composed of approximately 5 to 30 nucleotides, and that this sequence forms a specific bond with a protein having a specific amino acid sequence non-covalently, namely, via a hydrogen bond. Such a sequence forming a natural RNA-protein binding motif can be obtained by appropriately selecting a motif causing a desired structural change from the following Table 1 and Table 2, and also from the database available on the website: http://gibk26.bse.kyutech.ac.jp/jouhou/image/dna-protein/RNA/RNA.html. The RNA-protein binding motif preferably used in the present instance is a motif of which the structure has already been analyzed by X-ray crystal structural analysis or NMR, or a motif whose steric structure can be assumed from the steric structure of a homologous protein of which the structure has already been analyzed. Moreover, it is preferably an RNA-protein binding motif, in which the protein specifically recognizes the secondary structure and nucleotide sequence of the RNA.

**[Table 1]**

| Name of RNA | Name of protein | Kd | Publication |
|---|---|---|---|
| 5S RNA (*Xenopus laevis* oocyte) | 5R1 | 0.64 ± 0.10 nM | Nat Struct Biol. 1998 Jul;5(7):543-6 |
| 5S RNA (*Xenopus laevis* oocyte) | 5R2 | 0.35 ± 0.03 nM | Nat Struct Biol. 1998 Jul;5(7):543-6 |
| dsRNA | B2 | 1.4 ± 0.13 nM | Nat Struct Mol Biol. 2005 Nov; 12(11):952-7 |
| RNA splicing motif with UGCAUGU element | Fox-1 | 0.49 nM at 150 mM salt | EMBO J. 2006 Jan 11;25(1):163-73. |
| TGE | GLD-1 | 9.2 ± 2 nM | J Mol Biol. 2005 Feb 11;346(1):91-104. |
| sodB mRNA | Hfq | 1.8 nM | EMBO J. 2004 Jan 28;23(2):396-405. |
| RyhB (siRNA) | Hfq | 1500 nM | Ann Rev Microbiol. 2004;58:303-28 |
| mRNA | HuD | 0.7 ± 0.02 nM | Nat Struct Biol. 2001 Feb;8(2):141-5 |
| S domain of 7S RNA | human SRP19 | | RNA. 2005 Jul;11(7):1043-50. Epub 2005 May 31 |
| Large subunit of SRP RNA | human SRP19 | 2 nM | Nat Struct Biol. 2001 Jun;8(6):515-20 |
| 23S rRNA | L1 | | Nat Struct Biol. 2003 Feb; 10(2): 104-8 |
| 23S rRNA | L11 | | Nat Struct Biol. 2000 Oct;7(10):834-7 |
| 5S rRNA | L18 | | Biochem J. 2002 May 1;363(Pt 3):553-61 |
| 23S rRNA | L20 | 13 ± 2 nM | J Biol Chem. 2003 Sep 19;278(38):36522-30. |
| Own mRNA site 1 | L20 | 88 ± 23 nM | J Biol Chem. 2003 Sep 19;278(38):36522-30. |
| Own mRNA site2 | L20 | 63 ± 23 nM | Mol Microbiol. 2005 Jun;56(6):1441-56 |
| 23S rRNA | L23 | | J Biomol NMR. 2003 Jun;26(2):131-7 |
| 5S rRNA | L25 | | EMBO J. 1999 Nov 15; 18(22):6508-21 |
| Own mRNA | L30 | | Nat Struct Biol. 1999 Dec;6(12):1081-3. |
| mRNA | LicT | | EMBO J. 2002 Apr 15;21(8):1987-97 |
| Own mRNA | MS2 coat | 39 ± 5 nM | FEBS J. 2006 Apr;273(7):1463-75 |
| Stem-loop RNA motif | Nova-2 | | Cell. 2000 Feb 4;100(3):323-32 |
| SL2 | Nucleocapsid | 110 ± 50 nM | J Mol Biol. 2000 Aug 11;301(2):491-511 |
| Pre-rRNA | Nucleolin | | EMBO J. 2000 Dec 15;19(24):6870-81 |
| | p19 | 0.17 ± 0.02 nM | Cell. 2003 Dec 26;115(7):799-811 |
| Box C/D | L7Ae | 0.9 ± 0.2 nM | RNA. 2005 Aug;11(8):1192-200. |

**[Table 2]**

| Name of RNA | Name of protein | Kd | Publication |
|---|---|---|---|
| siRNA with the characteristic two-base 3' overhangs | PAZ(PiWi Argonaut and Zwille) | | Nat Struct Biol. 2003 Dec;10(12):1026-32. |
| dsRNA | Rnase III | | Cell. 2006 Jan 27;124(2):355-66 |
| HrV-1 RRE (IIB) | RR1-38 | 3-8 nM | Nat Struct Biol. 1998 Jul;5(7):543-6 |
| Own mRNA | S15 | 5 nM | EMBO J. 2003 Apr 15;22(8):1898-908 |
| 16S rRNA | S15 | 6 nM | Nat Struct Biol. 2000 Apr;7(4):273-277. |
| Own mRNA | S15 | 43 nM | EMBO J. 2003 Apr 15;22(8):1898-908 |
| 16S rRNA | S4 | 6.5 µM in 4°C, 1.7 nM in 42°C | J Biol Chem. 1979 Mar 25;254(6):1775-7 |
| 16S rRNA | S4 | 18 µM | J Biol Chem. 1979 Mar 25;254(6):1775-7 |
| 16S rRNA | S8 | 26 ± 7 nM | J Mol Biol. 2001 Aug 10;311(2):311-24 |
| mRNA | S8 | 200 nM | RNA. 2004 Jun;10(6):954-64 |
| mRNA | SacY | 1400 nM | EMBO J. 1997 Aug 15;16(16):5019-29 |
| SnRNA | Sm | | Cold Spring Harb Symp Quant Biol. 2006;71:313-20. |
| tmRNA | SmpB | 21 ± 7 nM | J Biochem (Tokyo). 2005 Dec;138(6):729-39 |
| TD3 of tmRNA | SmpB | 650 nM | J Biochem (Tokyo). 2005 Dec;138(6):729-39 |
| U1 snRNA | snRNP U1A | 0.032 ± 0.007 nM (salt dependence) | Nat Struct Biol. 2000 Oct;7(10):834-7 |
| S domain of 7S RNA | SRP54 | 500 nM | RNA. 2005 Jul;11(7):1043-50. |
| TAR | Tat | 200-800 nM | Nucleic Acids Res. 1996 Oct 15;24(20):3974-81 |
| BIV TAR | Tat | 1.3 nM or 8 nM or 60 nM (Changed depending on difference in Mg) | Mol Cell. 2000 Nov;6(5):1067-76 |
| tRNA^{Thr} | ThrRS | 500 nM | Nat Struct Biol. 2002 May;9(5):343-7 |
| *thrS* mRNA operator | ThrRS | 10 nM | Trends Genet. 2003 Mar;19(3):155-61 |
| Single stranded mRNA | TIS11d | | Nat Struct Mol Biol. 2004 Mar;11(3):257-64. |
| PSTVd | Virp1 | 500 nM | Nucleic Acids Res. 2003 Oct 1;31(19):5534-43 |
| RNA hairpin; Smaug recognition element (SRE) | Vts1p | 30 nM | Nat Struct Mol Biol. 2006 Feb;13(2):177-8. |
| λ BοxB | λ N | 90 nM | Cell. 1998 Apr 17;93(2):289-99 |

The RNA comprising a sequence forming an artificial RNA-protein binding motif means the side of RNA in an RNA-protein binding motif in an artificially designed RNA-protein complex. The nucleotide sequence of such RNA is generally composed of approximately 10 to 80 nucleotides, and such RNA is designed such that it forms a specific bond with a specific amino acid sequence of a specific protein non-covalently, namely, via a hydrogen bond. An example of such an RNA comprising a sequence forming an artificial RNA-protein binding motif is an RNA aptamer specifically binding to a specific protein. The RNA aptamer specifically binding to a desired protein as a target can be obtained, for example, by an evolution engineering method known as an *in vitro* selection method or a SELEX method. At this time, a trigger protein can be a protein to which the RNA aptamer binds. For example, RNA sequences shown in the following Table 3 are known, and these sequences can also be used as sequences forming the RNA-protein binding motif of the present disclosure.

**[Table 3]**

| Name of RNA | Name of protein | Kd | Publication |
|---|---|---|---|
| Rev aptamer 5 | Rev | 190 nM | RNA. 2005 Dec;11(12):1848-57 |
| Aptamer | p50 | 5.4 ± 2.2 nM | Proc Natl Acad Sci U S A. 2003 Aug 5;100(16):9268-73. |
| BMV Gag aptamer | BMV Gag | 20 nM | RNA. 2005 Dec;11(12):1848-57 |
| BMV Gag aptamer | CCMV Gag | 260 nM | RNA. 2005 Dec;11(12):1848-57 |
| CCMV Gag aptamer | CCMV Gag | 280 nM | RNA. 2005 Dec;11(12):1848-57 |
| CCMV Gag aptamer | BMV Gag | 480 nM | RNA. 2005 Dec;11(12):1848-57 |

In the present instance, the sequence forming an RNA-protein binding motif preferably has a dissociation constant Kd of approximately 0.1 nM to approximately 1 µM, for the corresponding protein.

In addition, not only the sequences forming these RNA-protein binding motifs, but mutants of these sequences are also included in the sequence according to the present disclosure. The term "mutant" is used in the present disclosure to mean a mutant having a dissociation constant Kd of 10%, 20%, 30%, 40%, 50% or more, or a mutant having a dissociation constant Kd of 10%, 20%, 30%, 40%, 50% or less, for a protein specifically binding to the sequence forming an RNA-protein binding motif. Such a mutant can be appropriately selected and used, as long as it is capable of forming an RNA-protein complex. Moreover, the nucleotide sequence of such a mutant may be a nucleotide sequence, which can be hybridized with a nucleic acid (complementary strand) having a sequence complementary to the sequence forming an RNA-protein binding motif (plus strand) under stringent conditions. Herein, the stringent conditions can be determined based on the melting temperature (Tm) of a nucleic acid, to which the nucleotide sequence binds, as taught in Berger and Kimmel (1987, Guide to Molecular Cloning Techniques Methods in Enzymology, Vol. 152, Academic Press, San Diego CA). For example, as washing conditions after completion of the hybridization, conditions such as "1 × SSC, 0.1% SDS, and 37°C" can be generally applied. The complementary strand preferably maintains a hybridized condition with the plus strand as a target, although it is washed under such conditions. Examples of more stringent hybridization conditions include, but are not particularly limited to, conditions under which the plus strand and the complementary strand maintain a hybridized condition, although they are washed under washing conditions such as "0.5 × SSC, 0.1% SDS, and 42°C," and more stringently, such as "0.1 × SSC, 0.1% SDS, and 65°C." Specifically, the present mutant consists of a nucleotide sequence having sequence identity of at least 90%, and preferably at least 95%, 96%, 97%, 98% or 99% with the sequence of RNA contained in the aforementioned RNA-protein binding motif. Such a mutant retains a constant bond with a protein specifically binding to a sequence forming an RNA-protein binding motif, and thereby, it can contribute to formation of an RNA-protein complex.

Specific examples of the sequence forming an RNA-protein binding motif according to the present instance include a box C motif (5'-GGCGUGAUGAGC-3') (SEQ ID NO: 1), a kink-loop (SEQ ID NO: 2), and a kink-loop 2 (SEQ ID NO: 3), to which L7Ae (Moore T et al., Structure Vol. 12, pp. 807-818 (2004)) binds.

Other specific examples include an MS2 stem loop motif that is a sequence to which an MS2 coat protein specifically binds (22: Keryer-Bibens C, Barreau C, Osborne HB (2008) Tethering of proteins to RNAs by bacteriophage proteins. Biol Cell 100: 125-138), and Fr15 that is a sequence to which a ribosome protein S15 of Bacillus binds (24: Batey RT, Williamson JR (1996) Interaction of the Bacillus stearothermophilus ribosomal protein S15 with 16 S rRNA: I. Defining the minimal RNA site. J Mol Biol 261: 536-549).

A further specific example is: 5'-GGCGUAUGUGAUCUUUCGUGUGGGUCACCACUGCGCC-3' (SEQ ID NO: 4) that is a sequence, to which Threonyl-tRNA synthetase (Cell (Cambridge, Mass.) v97, pp. 371-381 (1999)) binds that is known to be an enzyme performing aminoacylation and having feedback inhibition of binding to the mRNA thereof and inhibiting the translation thereof, and a mutant thereof. Further examples include R9-2 (5'-GGGUGCUUCGAGCGUAGGAAGAAAGCCGGGGGCUGCAGAUAAUGUAUAGC-3' (SEQ ID NO: 5)) that is a nucleotide sequence forming an RNA-protein binding motif derived from the Bcl-2 family CED-9 that is an endogenous protein specific to cancer cells, and a mutant thereof; and a nucleotide sequence derived from an aptamer of an RNA sequence binding to NF-kappa B, and a mutant thereof.

(ii) The sequences each consisting of 15 or more nucleotides, (iii) the 5'-terminal sequence, and (iv) the 3'-terminal sequence, which are included in the first RNA included in the first RNA-protein complex of the present disclosure, may be any given sequences. These sequences are preferably sequences, which do not comprise sequences complementary to one another and do not take a three-dimensional structure by the first RNA alone.

The "total length of (iii) the 5'-terminal sequence and (iv) the 3'-terminal sequence, which are included in the first RNA included in the first RNA-protein complex of the present disclosure, is 15 or more nucleotides" means that when the 5'-terminal sequence is linked to the 3'-terminal sequence, the length becomes 15 or more nucleotides. For example, it means that when the 5'-terminal sequence consists of one nucleotide, the 3'-terminal sequence consists of 14 or more nucleotides.

The first RNA included in the first RNA-protein complex of the present disclosure is configured, for example, in the order of (a 5'-terminal sequence) - (a sequence forming an RNA-protein binding motif) - [(a sequence consisting of 15 or more nucleotides) - (a sequence forming an RNA-protein binding motif)]ₙ- (a 3'-terminal sequence). Herein, the hyphen "-" indicates linking, and the phrase "[(a sequence consisting of 15 or more nucleotides) - (a sequence forming an RNA-protein binding motif)]ₙ" indicates that the thus-linked sequence is repeated n times. This number of repeats is, for example, 2 to 10, and the "[(a sequence consisting of 15 or more nucleotides) - (a sequence forming an RNA-protein binding motif)]" is preferably repeated two times, and specifically, the first RNA is one RNA having three sequences each forming an RNA-protein binding motif and two sequences each consisting of 15 or more nucleotides.

The sequence included in the second RNA included in the first RNA-protein complex of the present disclosure, which forms an RNA-protein binding motif together with the sequence included in the first RNA, is a sequence that supports formation of an RNA-protein binding motif, when the motif is formed. This sequence can be omitted depending on the RNA-protein binding motif selected, and it is, for example, a sequence comprising a sequence complementary to a portion of the RNA-protein binding motif. For example, when the sequence forming an RNA-protein binding motif, which is included in the first RNA, is a box C motif (SEQ ID NO: 1), this sequence is a box D motif (SEQ ID NO: 6), and in this case, the double-stranded RNA forming the RNA-protein binding motif may also be referred to as "K-turn."

The sequence included in the second RNA included in the first RNA-protein complex of the present disclosure, which is continuously complementary to the 5'-terminal sequence and the 3'-terminal sequence included in the first RNA, means a sequence complementary to an RNA sequence formed by linking the 5'-terminal sequence to the 3'-terminal sequence in the order of the 3'-terminal sequence - the 5'-terminal sequence. Since the 5'-terminal sequence and the 3'-terminal sequence included in the first RNA desirably consist of 15 or more nucleotides together, the complementary sequence included in the second RNA also desirably consists of 15 or more nucleotides.

The "5'-terminal sequence and the 3'-terminal sequence included in the second RNA included in the first RNA-protein complex of the present disclosure, which is continuously complementary to the sequence consisting of 15 or more nucleotides included in the first RNA," means that the RNA sequence formed by linking the 5'-terminal sequence to the 3'-terminal sequence in the order of the 3'-terminal sequence - the 5'-terminal sequence is complementary to the sequence consisting of 15 or more nucleotides included in the first RNA. Accordingly, the 5'-terminal sequence and the 3'-terminal sequence included in the second RNA desirably consist of 15 or more nucleotides, together.

The second RNA included in the first RNA-protein complex of the present disclosure is obtained, for example, by configuring a structure in the order of (a 5'-terminal sequence) - (a sequence forming an RNA-protein binding motif together with the first RNA) - [(a sequence complementary to the sequence consisting of 15 or more nucleotides included in the first RNA) - (a sequence forming an RNA-protein binding motif together with the first RNA)]ₙ - (a 3'-terminal sequence), and then by replacing any one (sequence complementary to the sequence consisting of 15 or more nucleotides included in the first RNA), with (a sequence continuously complementary to the 5'-terminal sequence and the 3'-terminal sequence included in the first RNA). The number of repeats, n, used for a portion enclosed in brackets [ ] in the second RNA has the same meaning as the number of repeats, n, used in the first RNA, and when the number of repeats is 2, the second RNA is one RNA having three sequences each forming an RNA-protein binding motif together with the first RNA and one sequence complementary to a sequence consisting of 15 or more nucleotides included in the first RNA.

With regard to proteins included in the first RNA-protein complex, if at least one protein is the fusion protein described in (3), the remaining two or more proteins may not be fusion proteins, as long as they are proteins each forming an RNA-protein binding motif. In the present disclosure, the fusion protein means a protein in which two or more types of proteins are operably linked to one another. The phrase "be operably linked" means that the proteins are linked to one another so that they function, and an example of such linking is the linking of proteins via a spacer sequence. The spacer sequence is preferably a sequence consisting of any given 2 to 200 amino acids. The amino acid sequence of such a spacer sequence is not particularly limited, as long as it does not affect the function of proteins contained in the fusion protein. An example of such an amino acid sequence is an amino acid sequence shown in SEQ ID NO: 28. The fusion protein described in (3) comprises at least a protein forming an RNA-protein binding motif. This protein can be selected, as appropriate, depending on the RNA in the RNA-protein binding motif. Examples of the fusion protein include, but are not particularly limited to, the proteins shown in Table 1, Table 2, and Table 3. In one instance of the present disclosure, when the RNA-protein binding motif is K-turn, the protein is, for example, L7Ae (Moore T et al., Structure Vol. 12, pp. 807-818 (2004)).

(3) At least one fusion protein included in the first RNA-protein complex of the present disclosure has the function of specifically recognizing a cell surface antigen, as well as the function of forming an RNA-protein binding motif. The fusion protein is, for example, a fusion protein of a protein forming an RNA-protein binding motif and a protein having the function of specifically recognizing a cell surface antigen.

In the present disclosure, the protein having the function of specifically recognizing a cell surface antigen is a protein specifically binding to a cell surface antigen, and examples of such a protein include an antibody, a ligand protein, and Affibody. Affibody is a protein produced by modifying a part of the IgG-binding domain of Protein A, and Affibody that binds to a desired cell surface antigen can be appropriately selected by screening. When the cell surface protein is HER2, examples of Affibody include (ZHER2:342)2 (Lina E., et al., PLOS ONE 2012, 7, 11, e49579) and Anti-ErbB2 Affibody Molecule (ab31889) (Abcam).

In the present disclosure, since the number of proteins included in an RNA-protein complex depends on the number of sequences each forming an RNA-protein binding motif included in the first RNA, it is possible to allow the RNA-protein complex to comprise three or more proteins. Among the three or more proteins, it may be adequate if at least one protein is the fusion protein described in (3), and the RNA-protein complex may comprise two or more fusion proteins described in (3). When the RNA-protein complex comprises two or more fusion proteins described in (3), these proteins may be (3) the fusion proteins of the same type, or may also be (3) the fusion proteins of different types. As a protein that forms an RNA-protein binding motif, a specific protein, which depends on and binds to a sequence forming an RNA-protein binding motif, can be used. Thus, it becomes possible to allow the RNA-protein complex to comprise a desired protein by appropriately changing the sequence forming an RNA-protein binding motif and by fusing a desired protein with the thus changed sequence. The protein included in the RNA-protein complex may optionally comprise one or two or more types of fusion proteins comprising a protein forming the RNA-protein binding motif and another functional protein.

In the present disclosure, examples of another functional protein include a membrane-permeable peptide, a signal peptide, an endosome release peptide, and a cytotoxic protein.

In the present disclosure, the fusion protein may be a protein comprising two or more types of other functional proteins having functions that are different from one another, and the fusion protein may be, for example, a fusion protein comprising a protein forming an RNA-protein binding motif, a protein specifically recognizing a cell surface antigen, and a membrane-permeable peptide. Alternatively, it may also be a fusion protein comprising the aforementioned proteins and a protein comprising an endosome release peptide.

In the present disclosure, examples of the membrane-permeable peptide include cell passage domains of proteins, such as fruit fly-derived AntP, HIV-derived TAT (Frankel, A. et al, Cell 55,1189-93 (1988) or Green, M. & Loewenstein, P. M. Cell 55, 1179-88 (1988)), Penetratin (Derossi, D. et al, J. Biol. Chem. 269, 10444-50 (1994)), Buforin II (Park, C. B. et al. Proc. Natl Acad. Sci. USA 97, 8245-50 (2000)), Transportan (Pooga, M. et al. FASEB J. 12, 67-77 (1998)), MAP (model amphipathic peptide) (Oehlke, J. et al. Biochim. Biophys. Acta. 1414, 127-39 (1998)), K-FGF (Lin, Y. Z. et al. J. Biol. Chem. 270, 14255-14258 (1995)), Ku70 (Sawada, M. et al. Nature Cell Biol. 5, 352-7 (2003)), Prion (Lundberg, P. et al. Biochem. Biophys. Res. Commun. 299, 85-90 (2002)), pVEC (Elmquist, A. et al. Exp. Cell Res. 269, 237-44 (2001)), Pep-1 (Morris, M. C. et al. Nature Biotechnol. 19, 1173-6 (2001)), Pep-7 (Gao, C. et al. Bioorg. Med. Chem. 10, 4057-65 (2002)), SynB1 (Rousselle, C. et al. Mol. Pharmacol. 57, 679-86 (2000)), HN-I (Hong, F. D. & Clayman, G L. Cancer Res. 60, 6551-6 (2000)), and HSV-derived VP22. By allowing the RNA-protein complex to comprise a membrane-permeable peptide, the RNA-protein complex is permeated through the cell membrane, so that it can be transported into the cell.

In the present disclosure, the signal peptide is an amino acid sequence associated with the transport or localization of a protein into a specific organelle in a cell, and examples of the organelle include a nucleus, a cytoplasm, an endoplasmic reticulum, a mitochondrion, a Golgi body, and peroxisome. A signal peptide to be transported or localized into a desired organelle whose function is to be exhibited can be selected, as appropriate. For instance, when the function of the after-mentioned siRNA included in the second RNA-protein complex is to be exhibited, a signal peptide capable of achieving cytoplasmic transport is preferable, and an example of such a signal peptide is a nuclear export signal (NES) (WO 2012/008361). Specific examples of the NES include, but are not limited to, peptides having the following amino acid sequences.

| | |
|---|---|
| LPPLERLTL | [SEQ ID NO: 7] (HIV-1 Rev protein-derived NES sequence) |
| ALQKKLEELELDE | [SEQ ID NO: 8] (MAPKK-derived NES sequence) |
| LALKLAGLDI | [SEQ ID NO: 9] (PKI-α-derived NES sequence) |
| SLEGAVSEISLRD | [SEQ ID NO: 10] (Dsk-1-derived NES sequence) |
| LPVLENLTL | [SEQ ID NO: 11] (TFIIIA-derived NES sequence) |
| LASLMNLGMS | [SEQ ID NO: 12] (Matrin3-derived NES sequence) |

In the present disclosure, examples of the cytotoxic protein include a protein inducing immune cells, a protein inducing cell death, and a protein forming a pore in a cell membrane. Examples of the protein inducing immune cells include an Fc site of an antibody and a complement. Examples of the protein inducing cell death include β-galactosidase, diphtheria toxin, Pseudomonas toxin, ricin, cholera toxin, a retinoblastoma gene, p53, herpes simplex thymidine kinase, varicella-zoster thymidine kinase, cytosine deaminase, nitroreductase, cytochrome p-4502 B1, thymidine phosphorylase, purine-nucleoside phosphorylase, alkaline phosphatase, carboxypeptidase A and G2, linamarase, β-lactamase, and xanthine oxidase. Examples of the protein forming a pore in a cell membrane include perforin, and parasporin produced by *Bacillus thuringiensis* A1470. By allowing the RNA-protein complex to comprise a cytotoxic protein, functions such as induction of immune cells, cell death, and formation of pores on the cell membrane can be exhibited in cells, to which the RNA-protein complex has been delivered.

In the present disclosure, the endosome release peptide is, for example, a peptide having such effects that a structure incorporated as a result of endocytosis is released from the endosome by the effects of this peptide, and the structure is then transferred to the cytoplasm, so as to avoid decomposition by the lysosome. Examples of this peptide include influenza virus hemagglutinin and GALA. By allowing the RNA-protein complex to comprise the endosome release peptide, the complex can be transferred to the cytoplasm. For example, when an RNA-protein complex to be delivered to a target cell is transported from the cell surface into the inside of the cell, and it is then trapped by the endosome, the RNA-protein complex can exhibit the function to knockdown the expression of the target gene by being transferred to the cytoplasm.

The upper limit of the sequence consisting of 15 or more nucleotides, which is included in the first RNA-protein complex of the present disclosure, is not particularly limited. The sequence consists of, for example, 150 or fewer nucleotides, 100 or fewer nucleotides, 92 or fewer nucleotides, 90 or fewer nucleotides, 80 or fewer nucleotides, 70 or fewer nucleotides, 60 or fewer nucleotides, 50 or fewer nucleotides, or 48 or fewer nucleotides, and it is more preferably a sequence consisting of 26 nucleotides.

The first RNA-protein complex of the present disclosure can be obtained by the following steps: a step of designing the first RNA, the second RNA and a fusion protein or a protein, which constitute the first RNA-protein complex, such that they comprise the above described molecular characteristics; a step of obtaining each of the first RNA, the second RNA and the fusion protein or the protein by a common genetic engineering method; and a step of mixing the first RNA, the second RNA and the fusion protein under conditions known to a person skilled in the art to form a complex. In the step of obtaining each of the first RNA, the second RNA and the fusion protein or the protein by a common genetic engineering method, each of the first RNA, the second RNA and the fusion protein or the protein can be obtained by an *in vitro* synthetic method using template DNA comprising a promoter sequence as a template. The step of forming a complex can be carried out by producing double-stranded RNA under conditions for hybridization with a known RNA strand, and then contacting the double-stranded RNA with the protein. The thus-formed first RNA-protein complex is used for carrying out an RNA and protein delivery system in an aqueous solution system.

It has been confirmed that, in the case of using a delivery system utilizing the first RNA-protein complex of the present disclosure, the cell recognition efficiency is increased when compared with a delivery system using a protein alone. Accordingly, in order to achieve more efficient delivery, RNA or a protein included in the RNA-protein complex can be selected, as appropriate. Preferably, by selecting, as appropriate, the length of the sequence of RNA, such cell recognition efficiency can be increased. The sequence of RNA can be determined by appropriately changing the sequence consisting of 15 or more nucleotides, the 5'-terminal sequence, and the 3'-terminal sequence, which are included in the RNA. In particular, the sequence of RNA can be determined by appropriately changing the number of nucleotides in the sequence consisting of 15 or more nucleotides, and the total number of nucleotides in the 5'-terminal sequence and the 3'-terminal sequence, all of which are included in the RNA. Such change can be carried out, particularly, from the viewpoint of enhancing the ability to bind to a cell surface antigen.

Accordingly, the first RNA-protein complex, in which the number of nucleotides has been appropriately changed, is used, and the ability of the first RNA-protein complex to bind to a desired cell surface antigen is then measured, so that the number of nucleotides that achieves high cell recognition ability can be determined. In this respect, according to one instance, the present disclosure can also include a method for selecting the nucleotide length of each of the sequences in (1) (ii), the sequences in (1) (iii), the sequence(s) in (2) (b), and the sequences in (2) (d), using the ability to bind to a cell surface antigen as an indicator, in the first RNA-protein complex.

Although, in general, RNA is easily decomposed in the presence of serum, the delivery system comprising the first RNA-protein complex of the present disclosure can be carried out in the presence of serum, because of the formation of the first RNA-protein complex. The serum is not particularly limited, and an example of such serum is serum of the same origin as that of the target cell in the present delivery system. Specific examples of the serum include fetal bovine serum and human serum. In one aspect of implementation of the present disclosure in the presence of serum, such serum is intravenously administered to a living body as a target of the first RNA-protein complex, for example.

Another instance of the RNA-protein complex of the present disclosure is a second RNA-protein complex comprising the following (1) to (4):
(1) a first RNA comprising the following sequences (i) to (iii):
   (i) a sequence forming an RNA-protein binding motif,
   (ii) two sequences each consisting of seven or more nucleotides, and
   (iii) a siRNA sequence;
(2) a second RNA comprising the following sequences (a) to (d):
   (a) a sequence forming an RNA-protein binding motif, together with (1) (i),
   (b) two or more sequences each consisting of 14 or more nucleotides, which are continuously complementary to the two sequences each consisting of seven or more nucleotides described in (1) (ii),
   (c) a 5'-terminal sequence, and
   (d) a 3'-terminal sequence, wherein
      (c) the 5'-terminal sequence and (d) the 3'-terminal sequence are continuously complementary to the sequences each consisting of seven or more nucleotides described in (1) (ii);
(3) at least one protein forming an RNA-protein binding motif with the RNAs formed by (1) (i) and (2) (a); and
(4) a third RNA comprising a sequence complementary to the siRNA sequence described in (1) (iii).

The sequence forming an RNA-protein binding motif included in the first RNA, and the sequence forming an RNA-protein binding motif together with the first RNA, which are used in the second RNA-protein complex of the present disclosure, can be the same as those used in the aforementioned first RNA-protein complex.

The sequence consisting of seven or more nucleotides included in the first RNA of the second RNA-protein complex of the present disclosure may be any given sequence. A preferred example of the sequence is a sequence, which does not comprise a sequence complementary to other sequences each consisting of seven or more nucleotides, and does not have a three-dimensional structure by the first RNA alone.

The siRNA sequence included in the first RNA of the second RNA-protein complex of the present disclosure is either one RNA sequence of double-stranded RNA constituting the siRNA, which is a portion of any given mRNA as a target of RNA interference. This is preferably a sequence consisting of 18 nucleotides to 35 nucleotides. Such a sequence can be searched for using search software provided free of charge on various websites. Examples of such websites include, but are not limited to, siRNA Target Finder (http://www.ambion.com/jp/techlib/misc/siRNA_finder.html) and Insert Design Tool for pSilencer™ Expression Vector
(http://www.ambion.com/jp/techlib/misc/psilencer_converter.html), both of which are provided by Ambion, and GeneSeer (http://codex.cshl.edu/scripts/newsearchhairpin.cgi) provided by RNAi Codex. Similar searching can be carried out on websites provided by QIAGEN, Takara Bio Inc., SiSearch, Dharmacon, Whitehead Institute, Invitrogen, Promega, etc.

The first RNA of the second RNA-protein complex of the present disclosure is configured, for example, in the order of 5' - (a siRNA sequence) - (a sequence consisting of seven or more nucleotides) - (a sequence forming an RNA-protein binding motif) - (a sequence consisting of seven or more nucleotides) - 3', or in the order of 5' - (a sequence consisting of seven or more nucleotides) - (a sequence forming an RNA-protein binding motif) - (a sequence consisting of seven or more nucleotides) - (an siRNA sequence) - 3'.

At least three first RNAs are preferably included in the second RNA-protein complex of the present disclosure, and the number of the first RNAs included in the second RNA-protein complex is, for example, 10, 9, 8, 7, 6, 5, 4 or the like. The number of the first RNAs included in the second RNA-protein complex is most preferably 3. The first RNAs included in the RNA-protein complex may have sequences that are different from one another, or may be composed of the same sequences. However, in order to avoid the binding of the first RNAs to one another, the first RNAs desirably do not comprise sequences that are complementary to one another.

The "sequences each consisting of 14 or more nucleotides that are continuously complementary to the two sequences each consisting of seven or more nucleotides included in the first RNA, which are included in the second RNA in the second RNA-protein complex of the present disclosure" mean sequences complementary to a sequence generated as a result of the linking of one sequence on the 3'-terminal side, among the sequences each consisting of seven or more nucleotides included in the first RNA, to another sequence on the 5'-terminal side, among other sequences each consisting of seven or more nucleotides included in the first RNA. The number of sequences each consisting of 14 or more nucleotides included in the second RNA, which are continuously complementary to the two sequences each consisting of seven or more nucleotides included in the first RNA, depends on the number of the first RNAs included in the second RNA-protein complex. When there are the three first RNAs, there are two sequences each consisting of 14 or more nucleotides included in the second RNA.

The "5'-terminal sequence and the 3'-terminal sequence included in the second RNA in the second RNA-protein complex of the present disclosure are continuously complementary to the sequences each consisting of seven or more nucleotides included in the first RNA" means that a portion of an RNA sequence formed by linking the 5'-terminal sequence to the 3'-terminal sequence in the order of the 3'-terminal sequence - the 5'-terminal sequence, which comprises both the 5'-terminal sequence and the 3'-terminal sequence, is complementary to the sequences each consisting of seven or more nucleotides included in the first RNA, and more preferably means that an RNA sequence consisting of the 3'-terminal sequence - the 5'-terminal sequence in this order is complementary to a sequence generated as a result of the linking of one sequence on the 3'-terminal side, among the sequences each consisting of seven or more nucleotides included in the first RNA, to another sequence on the 5'-terminal side, among other sequences each consisting of seven or more nucleotides included in the first RNA. Accordingly, the total length of the 3'-terminal sequence and the 5'-terminal sequence included in the second RNA becomes 14 or more nucleotides. For example, when the 3'-terminal sequence included in the second RNA consists of 13 nucleotides, the 5'-terminal sequence included in the second RNA consists of one or more nucleotides.

The second RNA included in the second RNA-protein complex of the present disclosure is obtained, for example, by configuring a structure in the order of (a 5'-terminal sequence) - (a sequence forming an RNA-protein binding motif together with the first RNA) - [(a sequence consisting of 14 or more nucleotides continuously complementary to the two sequences each consisting of seven or more nucleotides included in the first RNA) - (a sequence forming an RNA-protein binding motif together with the first RNA)]ₘ - (a 3'-terminal sequence), and then by replacing any one (sequence complementary to the sequences each consisting of 15 or more nucleotides included in the first RNA), with (a sequence continuously complementary to the 5'-terminal sequence and the 3'-terminal sequence included in the first RNA). The number of repeats, m, used for a portion enclosed in brackets [ ] in the second RNA means a number that is one less than the number of the first RNAs. Thus, when the number of repeats is 2, it means an RNA having three sequences each forming an RNA-protein binding motif together with the first RNA.

The protein included in the second RNA-protein complex of the present disclosure may be the same protein (3) or the same protein fusion, which are explained in the aforementioned first RNA-protein complex. Accordingly, the protein included in the RNA-protein complex according to one instance of the present disclosure may not only include a protein forming an RNA-protein binding motif, but also include a protein fusion comprising another functional protein.

Since the protein included in the second RNA-protein complex of the present disclosure depends on the number of the first RNAs, there are three or more types of proteins. Since a protein that is specific depending on the sequence forming an RNA-protein binding motif can be used as a protein forming an RNA-protein binding motif, it becomes possible to allow the RNA-protein complex to comprise a desired protein by appropriately changing the sequence forming an RNA-protein binding motif. For example, an RNA-protein complex, which comprises one type of fusion protein comprising a protein forming an RNA-protein binding motif and a protein specifically recognizing a cell surface antigen, and two or more types of fusion proteins comprising a protein forming an RNA-protein binding motif and another functional protein, can be formed.

The third RNA comprising a sequence complementary to the siRNA sequence, which is included in the RNA of the second RNA-protein complex of the present disclosure, is a sequence complementary to the siRNA sequence described in the aforementioned (1) (iii), wherein the sequence forms a double strand with the siRNA sequence to configure the siRNA, and thereby can exhibit its effects. Accordingly, the third RNA is preferably a sequence, in which two nucleotides on the 3'-terminal side does not form a complementary strand with the siRNA sequence described in (1) (iii). The same number of the third RNAs as that of the first RNAs is desirably included in the RNA-protein complex.

To the 5'-terminus of the first RNA or the 3'-terminus of the third RNA, which are included in the second RNA-protein complex of the present disclosure, a sequence promoting the transport of the complex to a cytoplasm may be further added, and an example of such a sequence promoting the transport of the complex to the cytoplasm is the sequence of tRNA. The tRNA is one of promoters recognizing RNA polymerase, and also, it acts as a cytoplasmic transport signal. Therefore, the tRNA is likely to support the second RNA-protein complex of the present disclosure in exhibiting the siRNA function (Japanese Patent No. 3831785).

Since the second RNA-protein complex of the present disclosure has the function of the siRNA included in the complex, it can be used as an RNA inhibitor. Moreover, the second RNA-protein complex can be used in a method of suppressing mRNA corresponding to a siRNA sequence in a cell by introducing the second RNA-protein complex into the cell. The second RNA-protein complex can be introduced into a cell in the same manner as the method of introducing the first RNA-protein complex into a cell. Such a method can also be referred to as a method of decomposing mRNA in a cell, comprising a step of contacting an RNA-protein complex with the cell. At this time, the contact with the cell can be carried out *in vivo*, or can also be carried out *in vitro.*

In one instance of the present disclosure, when the second RNA-protein complex comprises a fusion protein of a protein forming an RNA-protein binding motif and a protein specifically recognizing a cell surface antigen, it can be used in a delivery system. Such delivery can be carried out in the same aspect as that of the first RNA-protein complex. Hence, in order to achieve more efficient delivery, RNA or a protein included in the RNA-protein complex can be selected, as appropriate. Preferably, by appropriately determining the length of an RNA sequence, cell recognition ability can be increased. The change of the RNA sequence can be carried out by appropriately changing two sequences each consisting of seven or more nucleotides, a 5'-terminal sequence, and a 3'-terminal sequence, which are included in the RNA.

In particular, such change of the RNA sequence can be carried out by appropriately changing the number of nucleotides in the two sequences each consisting of seven or more nucleotides, and the total number of nucleotides in the 5'-terminal sequence and the 3'-terminal sequence, which are included in the RNA. Such change can be carried out, particularly, from the viewpoint of enhancing the ability to bind to a cell surface antigen. Accordingly, the second RNA-protein complex, in which the number of nucleotides has been appropriately changed, is used, and the ability of the second RNA-protein complex to bind to a desired cell surface antigen is then measured, so that the number of nucleotides that achieves high cell recognition ability can be determined. In this respect, according to one instance, the present disclosure can also include a method for selecting the nucleotide length of each of the sequences in (1) (ii), the sequence in (1) (iii), the sequences in (2) (b), and the sequence in (2) (d), using the ability to bind to a cell surface antigen as an indicator, in the second RNA-protein complex.

### [Examples]

Hereinafter, the present invention will be described more in detail in the following examples. However, the following examples are not intended to limit the scope of the present invention.

### [Example 1]

### Production of RNA-protein complex

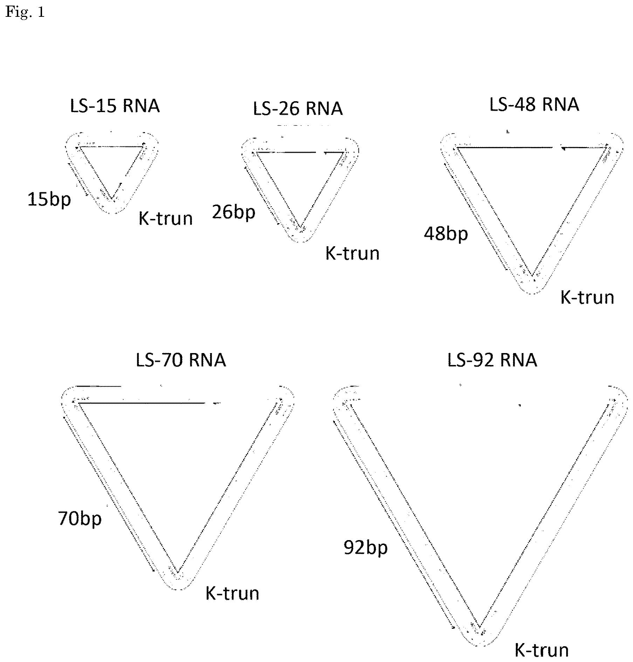

Triangular-structure RNA-protein complexes (Tri-RNPs, Figure 1) were produced in accordance with the description of Ohno, H. et al., Nat. Nanotechnol. 2011, 6, 116. Specifically, dsRNAs each consisting of a long chain (L-RNA) and a short chain (S-RNA), which had a K-turn RNA motif at a vertex thereof, and the side of each of which consisted of 15 nucleotides, 26 nucleotides, 48 nucleotides, 70 nucleotides or 92 nucleotides, were designed. The thus-produced RNA-protein complexes are referred to as LS-15 (L-15: SEQ ID NO: 13; and S-15: SEQ ID NO: 14), LS-26 (L-26: SEQ ID NO: 15; and S-26: SEQ ID NO: 16), LS-48 (L-48: SEQ ID NO: 17; and S-48: SEQ ID NO: 18), LS-70 (L-70: SEQ ID NO: 19; and S-70: SEQ ID NO: 20), and LS-92 (L-92: SEQ ID NO: 21; and S-92: SEQ ID NO: 22), respectively (Figure 1). RNA was produced based on the aforementioned DNA template, using MEGAshortscript T7 kit (Ambion). The obtained L-RNA and S-RNA were treated in an RNP-binding solvent (a solution consisting of 150 ml of KCl, 1.5 mM MgCl₂ and 20 mM HEPES-KOH (pH 7.5)) at 80°C for three minutes, and were then cooled at room temperature for 10 minutes, so that the RNAs were hybridized with each other to produce double-stranded RNA (LS-RNA) having a loop structure.

Thereafter, in accordance with the description of Ohno, H, et al., L7Ae was added to the LS-RNA, so as to form five triangular structures each having a different size. L7Ae was produced by transfecting a plasmid (a pET-28(+) vector (Novagen), into which the L7Ae sequence had been introduced) into *Escherichia coli* (Rosetta (DE3)/pLysSpRARE2).

The obtained five types of Tri-RNPs are referred to as Tri-15, Tri-26, Tri-48, Tri-70, and Tri-92. The sides of the Tri-RNPs are considered to have lengths of approximately 13.7, 16.7, 22.6, 28.5, and 34.4 nm, respectively.

Subsequently, according to electrophoretic mobility shift assay (EMSA), the interaction of L7Ae with the LS-RNA was analyzed. After completion of electrophoresis using polyacrylamide gel, the polyacrylamide gel was stained with SYBR Green I and II (Lonza), and formation of an RNA-protein complex was then observed. As a result, it was confirmed that a triangular structure was formed by the interaction of the LS-RNA with L7Ae, as expected, in all of the LS-RNAs (Figure 2).

Subsequently, the Tri-RNPs were observed by visual inspection. The Tri-RNPs (Tri-26 and Tri-48) were observed in a liquid phase, using high speed AFM (HS-AFM) (Nano Live Vision, Research Institute of Biomolecules Metrology) and a 10 × 2 × 0.1 µM cantilever (BL-AC10EGS, Olympus). The cantilever was set at a resonance frequency of 400-1000 kHz and at a spring constant of 0.1-0.2 N/m. A probe was deposited on the cantilever by electron beam. The mica surface was coated with 0.1% APTES, and thereafter, a 50 nM sample that had been dissolved in an RNP-binding solvent and an AFM observation solvent (20 mM Tris-HCl (pH7.6) and 10 mM MgCl2) was left at rest on the mica. The image was analyzed using software such as Image J and WSxM.

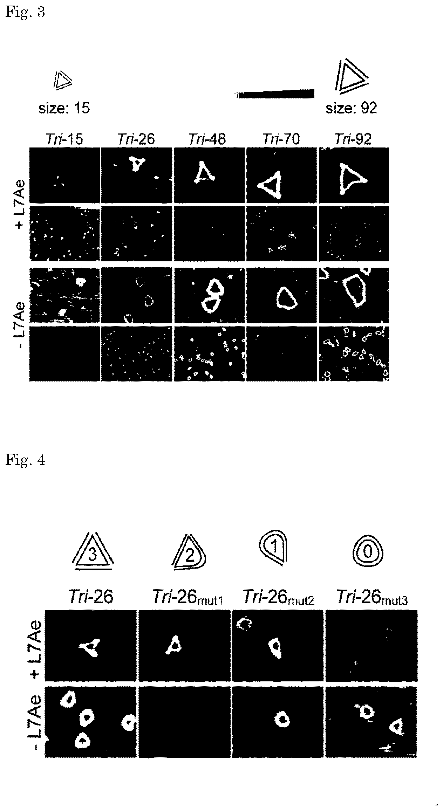

As a result, it was confirmed that when the LS-RNA was not contacted with L7Ae, the five LS-RNAs each having a different size each had a loop structure of double-stranded RNA. On the other hand, it was confirmed that when the LS-RNA was contacted with L7Ae, the five LS-RNAs each had a triangular structure. The state in which the RNA bound to a K-turn motif was confirmed in the form of a dot indicating a vertex of the Tri-RNP, and the inside of a triangle enclosed with three sides was confirmed to be a cavity. It was also confirmed that when the LS-RNA was contacted with L7Ae, it had a single structure (Figure 3). However, it was also confirmed that such a triangular structure was somewhat deformed in the case of Tri-92.

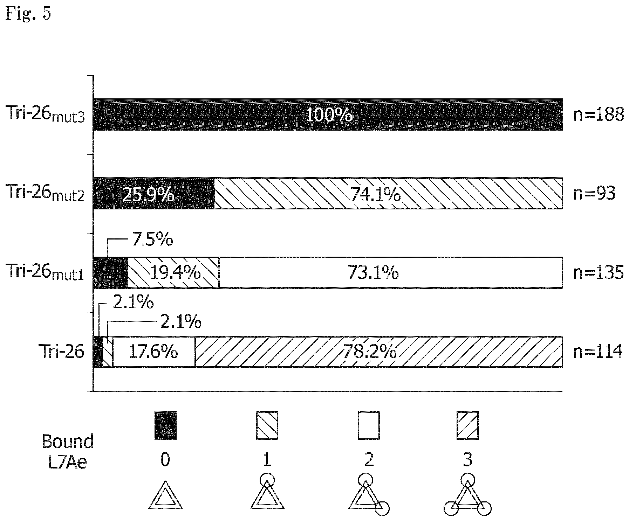

Subsequently, LS-RNAs (Tri-26ₘᵤₜ₁, Tri-26ₘᵤₜ₂ and Tri-26ₘᵤₜ₃) having one, two, or three mutant K-turn motifs (mut box C: SEQ ID NO: 23, mut box D: SEQ ID NO: 24) were examined according to EMSA. As a result, changes in the structures were found depending on the number of mutant K-turn motifs in each LS-RNA (Figure 4). At this time, individual structures (n = 114, 135, 93, or 188) were measured, and the ratio of structures formed was calculated. As a result, the content ratio of L7Ae and K-turn motifs was statistically matched with the ratio of the actually confirmed structures (Figure 5). From these results, it was confirmed that the interaction between three L7Ae and K-turn motifs was carried out at the vertex of a triangle.

### Detection of change in structure of RNA-protein complex (RNP), using time-lapse images

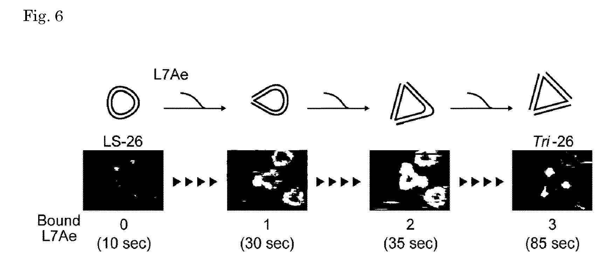

Using the aforementioned HS-AFM, a change in the structure of RNA after addition of L7Ae was observed by time-lapse imaging (Figure 6). From the time-lapse images, it was confirmed that when L7Ae directly binds to three K-turn motifs of LS-26, the K-turn motifs are bent at an angle of 60 degrees, and as a result of this structural change, the structure is thereby converted to a triangular structure. On the other hand, it was also confirmed that the triangular structure is converted to a loop structure by dissociation of a protein from RNA.

### [Example 2]

### Improvement of stability of RNA by interaction with protein

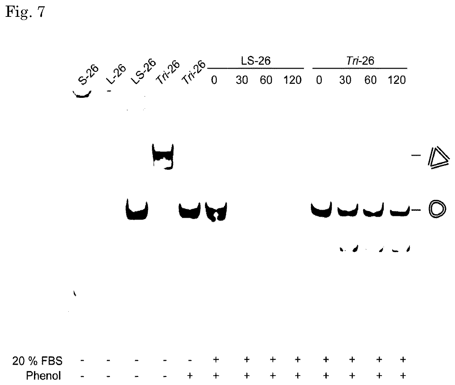

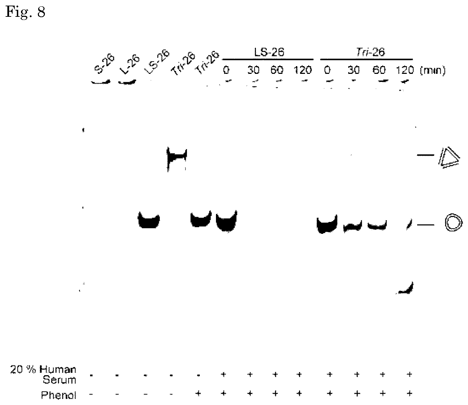

The structural stability of an RNA-protein complex (RNP) in the presence of serum was confirmed. Specifically, 20% FBS was added to 50 nM LS-26 or a mixture of 50 nM LS-26 and 300 nM L7Ae, and the obtained mixture was then incubated at 37°C for 0 minute, 30 minutes, 60 minutes, and 120 minutes. Thereafter, 10 mM EDTA (pH 8.0) and phenol were added to the reaction mixture, and the thus-obtained mixture was then electrophoresed using polyacrylamide gel, so that the stability of the RNA was observed. As a result, it was confirmed that, in the case of LS-26, the stability of RNA in a serum-containing buffer was promoted by forming RNP in the presence of L7Ae (Figure 7). On the other hand, it was also confirmed that LS-26 was decomposed by a treatment with serum for 30 minutes in the absence of L7Ae. Moreover, in the case of using Tri-26ₘᵤₜ₃, such decomposition was not suppressed. The same results were observed in the case of human serum (20%) (Figure 8). From these results, it was confirmed that the stability of RNA was also enhanced by forming a triangular structure even under physiological conditions such as in the presence of serum.

### [Example 3]

### Functional RNP having cell recognition ability

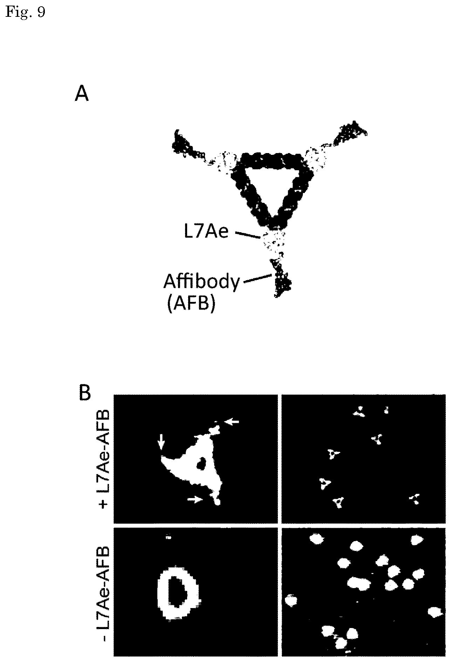

The function to recognize specific cancer cells was imparted to Tri-RNP. Specifically, a protein (L7Ae-AFB), in which the N-terminus of an affibody_{ZHER2:342} peptide (SEQ ID NOS: 25 and 26) was fused with the C-terminus of L7Ae via SSSGSSSGSSSG (SEQ ID NOS: 27 and 28), was prepared by transfecting a plasmid into *Escherichia coli* in accordance with Ohno, H., et al., as described above. This L7Ae-AFB was added to LS-RNA to construct RNP (Tri-AFB, Figure 9A). When LS-26 was used as RNA (Tri-26-AFB), a triangular structure was found by HS-AFM, and it was also confirmed that a substance assumed as AFB was added to each vertex (Figure 9B). Moreover, according to EMSA, it was confirmed that L7Ae-AFB binds to a K-turn motif, as with Tri-26 (Figure 10).

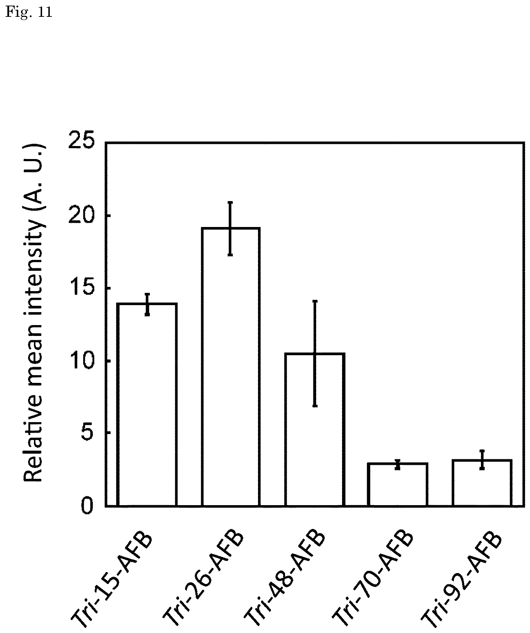

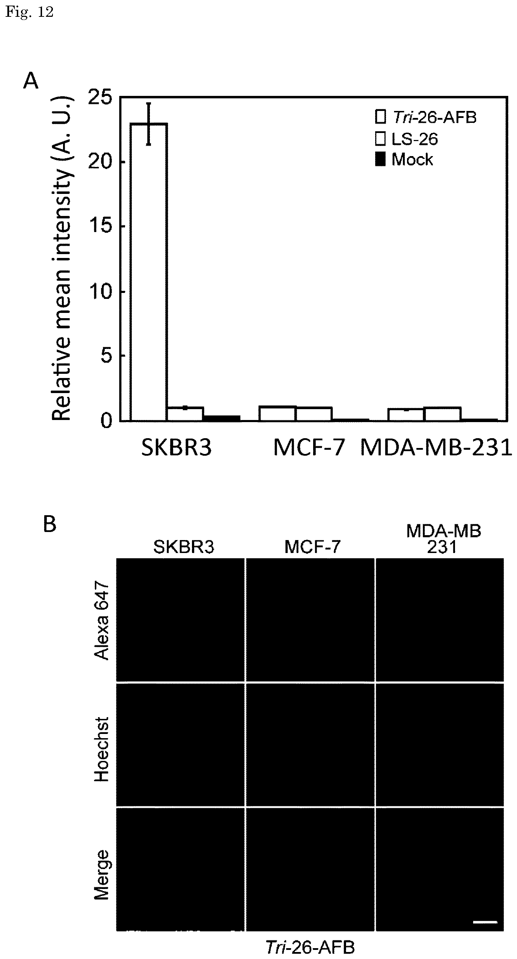

Subsequently, whether or not the Tri-AFB binds to breast cancer cells was examined. Specifically, a culture solution of the breast cancer cell line (SKBR3) (ATCC) was removed, and Opti-MEM was added. Thereafter, 10 nM LS-RNA or 10 nM Tri-AFB, which had been labeled with Alexa-647, was added, and the obtained mixture was then cultured at 37°C in 5% CO₂ for one hour. The binding of Tri-AFB to the cells after completion of the incubation was analyzed by a flow cytometer. As a result, Tri-26-AFB had the highest recognition ability (Figure 11). In order to examine the selective recognition ability of Tri-26-AFB, three different breast cancer cell lines (SKBR3 (HER2-strongly-positive line), MCF-7 (HER2-weakly-positive line), and MDA-MB-231 (HER2-negative line)) were used, and the ability of Tri-26-AFB was examined in the same manner as described above. As a result, it was confirmed that Tri-26-AFB selectively recognizes SKBR3, as expected (Figure 12).

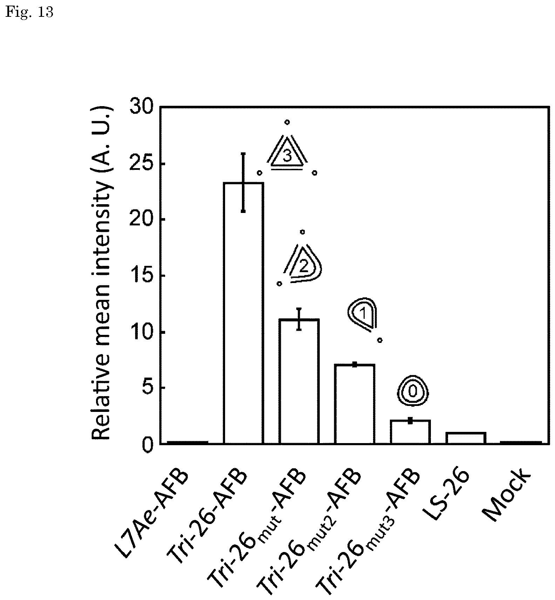

Furthermore, Tri-26 mutants having a mutant K-turn motif (Tri-26ₘᵤₜ₁, Tri-26ₘᵤₜ₂ and Tri-26ₘᵤₜ₃) were also examined in the same manner as described above. As a result, it was confirmed that the ability of these Tri-26 mutants to bind to SKBR3 is decreased depending on the number of mutant K-turn motifs (Figure 13). This suggests that an increase in the number of binding of AFB to HER2 influences recognition ability.

From these results, it was suggested that RNA can be transported onto the surface of a target cell by using Tri-AFB. However, it was also confirmed that RNP having the ability to bind to cells has an optimal size.

### [Example 4]

### Functional RNP having siRNA function

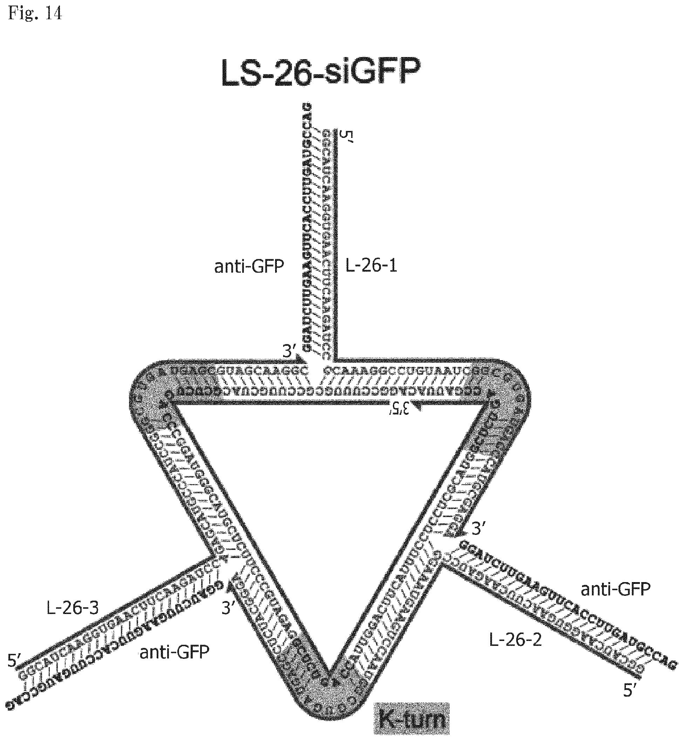

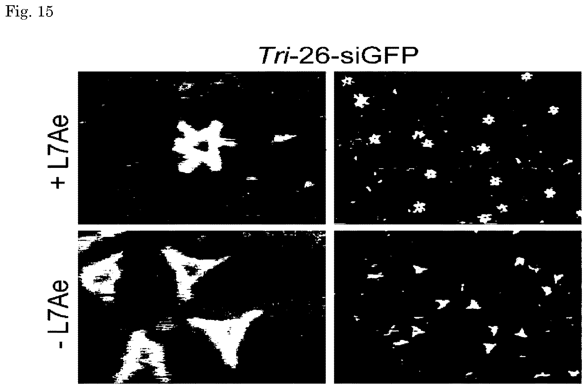

In order to study whether RNP having a triangular structure has a function as siRNA, the RNP shown in Figure 14 was constructed. Specifically, five RNAs were hybridized with S-26, L-26-1 (SEQ ID NO: 29), L-26-2 (SEQ ID NO: 30), L-26-3 (SEQ ID NO: 31), and antisense-GFP (SEQ ID NO: 32) in the same manner as described above, such that L-26 had siRNA for GFP at the three sides thereof, thereby producing LS-26-siGFP. Subsequently, LS-26-siGFP was mixed with L7Ae to produce Tri-26-siGFP having a triangular structure, and the structure of the Tri-26-siGFP was then confirmed by HS-AFM. As a result, a structure assumed to be siRNA for GFP was found at each side of the triangle, and it was confirmed that the Tri-26-siGFP had an assumed shape (upper pictures of Figure 15). On the other hand, it was confirmed that the Tri-26-siGFP had a heterogeneous RNA structure in the absence of L7Ae (lower pictures of Figure 15). As given above, a triangular structure (Tri-26-siGFP) having a structure containing three double strands having siRNA function could be constructed.

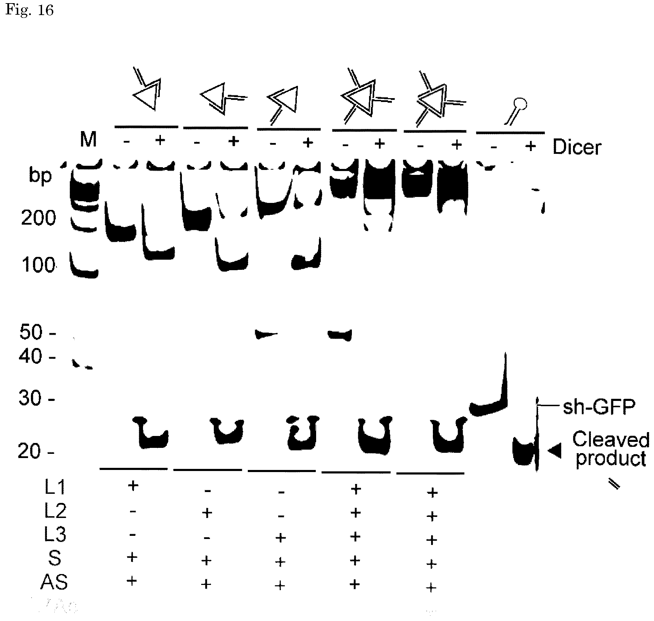

Whether this Tri-26-siGFP is involved in substrate recognition by Dicer was studied. Specifically, 500 nM LS-26-siGFPs (RNA hybridizing with L-26-1, S-26 and antisense-GFP, RNA hybridizing with L-26-2, S-26 and antisense-GFP, RNA hybridizing with L-26-3, S-26 and antisense-GFP, and LS-26-siGFP) or a mixture of 500 nM LS-26-siGFP and 3 µM L7Ae, was added to 1 µL of 10 mM ATP, 0.5 µL of 50 mM MaCl₂, 4 µL of Dicer Reaction buffer, and 1 µL of recombinant Dicer (Genlantis), and thereafter, water was added to the mixture to 10 µL. The thus obtained mixture was incubated at 37°C for 16 hours. After completion of the incubation, Dicer Stop Solution was added to the reaction mixture, and RNA cleaved with the Dicer, and RNA which had not been cleaved with the Dicer and remained, were confirmed by polyacrylamide gel electrophoresis. As a result, it was confirmed that Tri-26-siGFP contributes to the cleavage of RNA with Dicer, as with sh-GFP (SEQ ID NO: 33) used as a control (Figure 16). Accordingly, it was suggested that even Tri-26-siGFP having a triangular structure as a result of the interaction of L7Ae with a K-turn motif has the effect of siRNA without steric hindrance.

As described above, it was suggested that RNP having a triangular structure stabilizes RNA and is able to impart functions such as cell recognition ability, decomposition of mRNA by siRNA, and inhibition, to the RNA.

Subsequently, the knockdown activity of Tri-26-siGFP in cells was examined. 1 nM and 5 nM Tri-26-siGFP, 1 nM and 5 nM LS-26-siGFP, and 3 nM and 15 nM sh-GFP used as a positive control, were each transfected into HeLa cells stably expressing GFP (HeLa-GFP). The knockdown activity on GFP was measured based on the expression level thereof by flow cytometry. As a result, it was demonstrated that both Tri-26-siGFP and LS-26-siGFP could knock GFP down at an activity that is approximately 3 times higher than that of sh-GFP.

## Claims

1. An RNA-protein complex comprising the following (1) to (4):
(1) an RNA comprising the following sequences (i) to (iii):
(i) a sequence forming an RNA-protein binding motif,
(ii) two sequences each consisting of seven or more nucleotides, and
(iii) a siRNA sequence;
(2) an RNA comprising the following sequences (a) to (d):
(a) a sequence forming an RNA-protein binding motif, together with (1) (i),
(b) two or more sequences each consisting of 14 or more nucleotides, which are continuously complementary to the two sequences each consisting of seven or more nucleotides described in (1) (ii),
(c) a 5'-terminal sequence, and
(d) a 3'-terminal sequence, wherein
(c) the 5'-terminal sequence and (d) the 3'-terminal sequence are continuously complementary to the sequences each consisting of seven or more nucleotides described in (1) (ii);
(3) at least one protein forming an RNA-protein binding motif with the RNAs formed by (1) (i) and (2) (a); and
(4) an RNA comprising a sequence complementary to the siRNA sequence described in (1) (iii).

2. The RNA-protein complex according to claim 1, wherein there are the three RNAs described in (1), there are the two sequences each consisting of 14 or more nucleotides described in (2) (b), and there are the three RNAs described in (4).

3. The RNA-protein complex according to claim 1 or 2, wherein the sequence described in (1) (ii) consists of 7 or more nucleotides and 85 or fewer nucleotides, and the sequence described in (2) (b) consists of 14 or more nucleotides and 92 or fewer nucleotides.

4. The RNA-protein complex according to any one of claims 1 to 3, wherein the RNA-protein binding motifs formed by (1) (i) and (2) (a) are K-turn motifs, and the protein described in (3) is L7Ae.

5. The RNA-protein complex according to any one of claims 1 to 4, wherein the protein described in (3) is a fusion protein further comprising a protein specifically recognizing a cell surface antigen or a membrane-permeable peptide.

6. The RNA-protein complex according to claim 5, wherein the membrane-permeable peptide is a peptide selected from the group consisting of AntP, HIV-derived TAT, Penetratin, Buforin II, Transportan, MAP, K-FGF, Ku70, Prion, pVEC, Pep-1, Pep-7, SynB1, HN-I, and HSV-derived VP22.

7. A method for decomposing mRNA in cells, which comprises a step of contacting the RNA-protein complex according to any one of claims 1 to 6 with cells.

8. A method for selecting the nucleotide length of each of the sequences in the following (1) (ii) and the sequences in the following (2) (b), using the ability to bind to a cell surface antigen as an indicator, in an RNA-protein complex comprising the following (1) to (4):
(1) an RNA comprising the following sequences (i) to (iii):
(i) a sequence forming an RNA-protein binding motif,
(ii) two sequences each consisting of seven or more nucleotides, and
(iii) a siRNA sequence;
(2) an RNA comprising the following sequences (a) to (d):
(a) a sequence forming an RNA-protein binding motif, together with (1) (i),
(b) two or more sequences each consisting of 14 or more nucleotides, which are continuously complementary to the two sequences each consisting of seven or more nucleotides described in (1) (ii),
(c) a 5'-terminal sequence, and
(d) a 3'-terminal sequence, wherein
(c) the 5'-terminal sequence and (d) the 3'-terminal sequence are continuously complementary to the sequences each consisting of seven or more nucleotides described in (1) (ii);
(3) at least one fusion protein comprising a protein forming an RNA-protein binding motif with the RNAs formed by (1) (i) and (2) (a) and a protein specifically recognizing a cell surface antigen; and
(4) an RNA comprising a sequence complementary to the siRNA sequence described in (1) (iii).

## Patentansprüche

1. RNA-Protein-Komplex, beinhaltend die folgenden (1) bis (4):
(1) eine RNA, beinhaltend die folgenden Sequenzen (i) bis (iii):
(i) eine Sequenz, die ein RNA-Protein-Bindungsmotiv bildet,
(ii) zwei Sequenzen, die jeweils aus sieben oder mehr Nukleotiden bestehen, und
(iii) eine siRNA-Sequenz;
(2) eine RNA, beinhaltend die folgenden Sequenzen (a) bis (d):
(a) eine Sequenz, die ein RNA-Protein-Bindungsmotiv bildet, zusammen mit (1) (i),
(b) zwei oder mehr Sequenzen, die jeweils aus 14 oder mehr Nukleotiden bestehen, die zu den zwei in (1) (ii) beschriebenen Sequenzen, die jeweils aus sieben oder mehr Nukleotiden bestehen, kontinuierlich komplementär sind,
(c) eine 5'-Terminal-Sequenz und
(d) eine 3'-Terminal-Sequenz, wobei
(c) die 5'-Terminal-Sequenz und (d) die 3'-Terminal-Sequenz zu den in (1) (ii) beschriebenen Sequenzen, die jeweils aus sieben oder mehr Nukleotiden bestehen, kontinuierlich komplementär sind;
(3) mindestens ein Protein, das ein RNA-Protein-Bindungsmotiv mit den durch (1) (i) und (2) (a) gebildeten RNAs bildet; und
(4) eine RNA, die zu der in (1) (iii) beschriebenen siRNA-Sequenz kontinuierlich komplementär ist.

2. RNA-Protein-Komplex gemäß Anspruch 1, wobei die drei in (1) beschriebenen RNAs vorliegen, die zwei in (2) (b) beschriebenen Sequenzen, die jeweils aus 14 oder mehr Nukleotiden bestehen, vorliegen und die drei in (4) beschriebenen RNAs vorliegen.

3. RNA-Protein-Komplex gemäß Anspruch 1 oder 2, wobei die in (1) (ii) beschriebene Sequenz aus 7 oder mehr Nukleotiden besteht und 85 oder weniger Nukleotiden besteht und die in (2) (b) beschriebene Sequenz aus 14 oder mehr Nukleotiden und 92 oder weniger Nukleotiden besteht.

4. RNA-Protein-Komplex gemäß einem der Ansprüche 1 bis 3, wobei die durch (1) (i) und (2) (a) gebildeten RNA-Protein-Bindungsmotive K-turn-Motiv sind und das in (3) beschriebene Protein L7Ae ist.

5. RNA-Protein-Komplex gemäß einem der Ansprüche 1 bis 4, wobei das in (3) beschriebene Protein ein Fusionsprotein ist, ferner beinhaltend ein Protein, das spezifisch ein Zelloberflächenantigen erkennt, oder ein membranpermeables Peptid.

6. RNA-Protein-Komplex gemäß Anspruch 5, wobei das membranpermeable Peptid ein Peptid ist, das aus der Gruppe ausgewählt ist, die aus AntP, HIV-derived TAT, Penetratin, Buforin II, Transportan, MAP, K-FGF, Ku70, Prion, pVEC, Pep-1, Pep-7, SynB1, HN-I und HSV-derived VP22 besteht.

7. Verfahren zum Zerlegen von mRNA in Zellen, das einen Schritt des Kontaktierens des RNA-Protein-Komplexes gemäß einem der Ansprüche 1 bis 6 mit Zellen beinhaltet.

8. Verfahren zum Auswählen der Nukleotidlänge von jeder der Sequenzen in den folgenden (1) (ii) und der Sequenzen in den folgenden (2) (b), unter Verwendung der Fähigkeit, ein Zelloberflächenantigen als Indikator zu binden, in einem RNA-Protein-Komplex, beinhaltend die folgenden (1) bis (4):
(1) eine RNA, beinhaltend die folgenden Sequenzen (i) bis (iii):
(i) eine Sequenz, die ein RNA-Protein-Bindungsmotiv bildet,
(ii) zwei Sequenzen, die jeweils aus sieben oder mehr Nukleotiden bestehen, und
(iii) eine siRNA-Sequenz;
(2) eine RNA, beinhaltend die folgenden Sequenzen (a) bis (d):
(a) eine Sequenz, die ein RNA-Protein-Bindungsmotiv bildet, zusammen mit (1) (i),
(b) zwei oder mehr Sequenzen, die jeweils aus 14 oder mehr Nukleotiden bestehen, die zu den zwei in (1) (ii) beschriebenen Sequenzen, die jeweils aus sieben oder mehr Nukleotiden bestehen, kontinuierlich komplementär sind,
(c) eine 5'-Terminal-Sequenz und
(d) eine 3'-Terminal-Sequenz, wobei
(c) die 5'-Terminal-Sequenz und (d) die 3'-Terminal-Sequenz zu den in (1) (ii) beschriebenen Sequenzen, die jeweils aus sieben oder mehr Nukleotiden bestehen, kontinuierlich komplementär sind;
(3) mindestens ein Fusionsprotein, beinhaltend ein Protein, das ein RNA-Protein-Bindungsmotiv mit den durch (1) (i) und (2) (a) gebildeten RNAs bildet, und ein Protein, das spezifisch ein Zelloberflächenantigen erkennt; und
(4) eine RNA, beinhaltend eine Sequenz, die zu der in (1) (iii) beschriebenen siRNA-Sequenz komplementär ist.

## Revendications

1. Complexe ARN-protéine comprenant les aspects (1) à (4) suivants :
(1) un ARN comprenant les séquences (i) à (iii) suivantes :
(i) une séquence formant un motif de liaison ARN-protéine,
(ii) deux séquences, chacune étant constituée de sept nucléotides ou plus, et
(iii) une séquence ARNsi ;
(2) un ARN comprenant les séquences (a) à (d) suivantes :
(a) une séquence formant un motif de liaison ARN-protéine, ensemble avec (1) (i),
(b) deux séquences ou plus, chacune étant constituée de 14 nucléotides ou plus, qui sont complémentaires de manière continue aux deux séquences, chacune étant constituée de sept nucléotides ou plus, décrites en (1) (ii),
(c) une séquence terminale 5', et
(d) une séquence terminale 3', dans lequel
(c) la séquence terminale 5' et (d) la séquence terminale 3' sont complémentaires de manière continue aux séquences, chacune étant constituée de sept nucléotides ou plus, décrites en (1) (ii) ;
(3) au moins une protéine formant un motif de liaison ARN-protéine avec les ARN formés par (1) (i) et (2) (a) ; et
(4) un ARN comprenant une séquence complémentaire à la séquence ARNsi décrite en (1) (iii).

2. Complexe ARN-protéine selon la revendication 1, dans lequel les trois ARN sont décrits en (1), les deux séquences, chacune étant constituée de 14 nucléotides ou plus, sont décrites en (2) (b), et les trois ARN sont décrits en (4).

3. Complexe ARN-protéine selon la revendication 1 ou la revendication 2, dans lequel la séquence décrite en (1) (ii) est constituée de 7 nucléotides ou plus et de 85 nucléotides ou moins, et la séquence décrite en (2) (b) est constituée de 14 nucléotides ou plus et de 92 nucléotides ou moins.

4. Complexe ARN-protéine selon l'une quelconque des revendications 1 à 3, dans lequel les motifs de liaison ARN-protéine formés par (1) (i) et (2) (a) sont des motifs K-turn, et la protéine décrite en (3) est la L7Ae.

5. Complexe ARN-protéine selon l'une quelconque des revendications 1 à 4, dans lequel la protéine décrite en (3) est une protéine de fusion comprenant en outre une protéine reconnaissant spécifiquement un antigène de surface cellulaire ou un peptide perméable aux membranes.

6. Complexe ARN-protéine selon la revendication 5, dans lequel le peptide perméable aux membranes est un peptide choisi dans le groupe constitué par AntP, TAT dérivé du VIH, Pénétratine, Buforine II, Transportane, MAP, K-FGF, Ku70, Prion, pVEC, Pep-1, Pep-7, SynB1, HN-I, et VP22 dérivé de VHS.

7. Procédé de décomposition d'ARNm dans les cellules, qui comprend une étape de mise en contact du complexe ARN-protéine selon l'une quelconque des revendications 1 à 6 avec des cellules.

8. Procédé de sélection de la longueur de nucléotide de chacune des séquences dans la (1) (ii) suivante et des séquences dans la (2) (b) suivante, en utilisant la capacité à se lier à un antigène de surface cellulaire en tant qu'indicateur, dans un complexe ARN-protéine comprenant les aspects (1) à (4) suivants :
(1) un ARN comprenant les séquences (i) à (iii) suivantes :
(i) une séquence formant un motif de liaison ARN-protéine,
(ii) deux séquences, chacune étant constituée de sept nucléotides ou plus, et
(iii) une séquence ARNsi ;
(2) un ARN comprenant les séquences (a) à (d) suivantes :
(a) une séquence formant un motif de liaison ARN-protéine, ensemble avec (1) (i),
(b) deux séquences ou plus, chacune étant constituée de 14 nucléotides ou plus, qui sont complémentaires de manière continue aux deux séquences, chacune étant constituée de sept nucléotides ou plus, décrites en (1) (ii),
(c) une séquence terminale 5', et
(d) une séquence terminale 3', dans lequel
(c) la séquence terminale 5' et (d) la séquence terminale 3' sont complémentaires de manière continue aux séquences, chacune étant constituée de sept nucléotides ou plus, décrites en (1) (ii) ;
(3) au moins une protéine de fusion comprenant une protéine formant un motif de liaison ARN-protéine avec les ARN formés par (1) (i) et (2) (a) et une protéine reconnaissant spécifiquement un antigène de surface cellulaire ; et
(4) un ARN comprenant une séquence complémentaire à la séquence ARNsi décrite en (1) (iii).


### SEQUENCE LISTING

<110> Kyoto University
<120> RNA-protein complex and delivery system for RNA and protein thereby
<130> 2654-PCT
<150> JP2014-141431
   <151> 2014-07-09
<160> 33
<170> PatentIn version 3.5
<210> 1
   <211> 12
   <212> RNA
   <213> Artificial Sequence
<220>
   <223> fragment
<400> 1
   ggcgugauga gc 12
<210> 2
   <211> 34
   <212> RNA
   <213> Artificial Sequence
<220>
   <223> fragment
<400> 2
   agauccgggu gugaacggug aucacccgag aucc 34
<210> 3
   <211> 45
   <212> RNA
   <213> Artificial Sequence
<220>
   <223> fragment
<400> 3
   agauccggac guacguguga acggugauca cguacgccga gaucc 45
<210> 4
   <211> 37
   <212> RNA
   <213> Artificial Sequence
<220>
   <223> fragment
<400> 4
   ggcguaugug aucuuucgug ugggucacca cugcgcc 37
<210> 5
   <211> 50
   <212> RNA
   <213> Artificial Sequence
<220>
   <223> fragment
<400> 5
   gggugcuucg agcguaggaa gaaagccggg ggcugcagau aauguauagc 50
<210> 6
   <211> 9
   <212> RNA
   <213> Artificial Sequence
<220>
   <223> fragment
<400> 6
   gcucugacc 9
<210> 7
   <211> 9
   <212> PRT
   <213> Artificial Sequence
<220>
   <223> fragment
<400> 7
   Leu Pro Pro Leu Glu Arg Leu Thr Leu
   1               5
<210> 8
   <211> 13
   <212> PRT
   <213> Artificial Sequence
<220>
   <223> fragment
<400> 8
   Ala Leu Gln Lys Lys Leu Glu Glu Leu Glu Leu Asp Glu
   1 5 10
<210> 9
   <211> 10
   <212> PRT
   <213> Artificial Sequence
<220>
   <223> fragment
<400> 9
   Leu Ala Leu Lys Leu Ala Gly Leu Asp Ile
   1 5 10
<210> 10
   <211> 13
   <212> PRT
   <213> Artificial Sequence
<220>
   <223> fragment
<400> 10
   Ser Leu Glu Gly Ala Val Ser Glu Ile Ser Leu Arg Asp
   1 5 10
<210> 11
   <211> 9
   <212> PRT
   <213> Artificial Sequence
<220>
   <223> fragment
<400> 11
   Leu Pro Val Leu Glu Asn Leu Thr Leu
   1 5
<210> 12
   <211> 10
   <212> PRT
   <213> Artificial Sequence
<220>
   <223> fragment
<400> 12
   Leu Ala Ser Leu Met Asn Leu Gly Met Ser
   1 5 10
<210> 13
   <211> 100
   <212> RNA
   <213> Artificial Sequence
<220>
   <223> fragment
<400> 13
   ggugguaugc ucaucacgcc cagggccggc acagggcuca ucacgccgaa gccggacuuc      60
   ucgcucauca cgcccuucac cuauagugag ucguauuagc                            100
<210> 14
   <211> 91
   <212> RNA
   <213> Artificial Sequence
<220>
   <223> fragment
<400> 14
   ggcuucgguc agagcccugu gccggcccug ggucagagca uuccaccggu gaagggucag       60
   agcgagaagu ccuauaguga gucguauuag c                                     91
<210> 15
   <211> 114
   <212> RNA
   <213> Artificial Sequence
<220>
   <223> fragment
<400> 15
   ggcgcaaagg ccuguaaucg gcgugaugag ccaugcgagg aggaaaugaa guccaauggc       60
   gugaugagcc ucuacgggaa gagcaugccc auccgggcgu gaugagcgua gcaa           114
<210> 16
   <211> 105
   <212> RNA
   <213> Artificial Sequence
<220>
   <223> fragment
<400> 16
   ggccuuugcg ccuugcuacg cucugacccg gaugggcaug cucuucccgu agaggcucug       60
   accauuggac uucauuuccu ccucgcaugg cucugaccga uuaca                     105
<210> 17
   <211> 180
   <212> RNA
   <213> Artificial Sequence
<220>
   <223> fragment
<400> 17
   ggacgagcug uacaccaugg ugaccgccgc cgggcgugau gagcuccaag gaccccaacg        60
   agaagcgcga ucacaugauc uacuucggcu ucggcgugau gagcccugug cugcugcccg       120
   auaaccacua ccugccauca cccacggccc ugggcgugau gagcauucca cccagagcgc       180
<210> 18
   <211> 171
   <212> RNA
   <213> Artificial Sequence
<220>
   <223> fragment
<400> 18
   gguguacagc ucguccgcgc ucugggugga augcucugac ccagggccgu gggugauggc        60
   agguaguggu uaucgggcag cagcacaggg cucugaccga agccgaagua gaucauguga       120
   ucgcgcuucu cguugggguc cuuggagcuc ugacccggcg gcggucacca u               171
<210> 19
   <211> 246
   <212> RNA
   <213> Artificial Sequence
<220>
   <223> fragment
<400> 19
   ggacgagcug uacaccacac ggacaugacc accaaaacuc uggcgugaug agcucuuuua        60
   cggugcugau ggggagcagg ucugacccgc acgcacgcgg agggaccgca ggcccuuuca       120
   ggaggcguga ugagccaagc guggcgauag guaccaauau aagcgggcgg cugccaugua       180
   aucaccaacg gauaauuugc gguuuggcgu gaugagcuug aggucaacgu caagguuucg       240
   cggugc                                                                  246
<210> 20
   <211> 237
   <212> RNA
   <213> Artificial Sequence
<220>
   <223> fragment
<400> 20
   ggugaacagc ucguccgcac cgcgaaaccu ugacguugac cucaagcucu gaccaaaccg 60
   caaauuaucc guuggugauu acauggcagc cgcccgcuua uauugguacc uaucgccacg 120
   cuuggcucug accuccugaa agggccugcg gucccuccgc gugcgugcgg gucagaccug 180
   cuccccauca gcaccguaaa agagcucuga ccagaguuuu gguggucaug uccgugu 237
<210> 21
   <211> 312
   <212> RNA
   <213> Artificial Sequence
<220>
   <223> fragment
<400> 21
   ggacgagcug uacaccacac ggacaugacc caaccaauua caccaaaacu cuggcgugau 60
   gagcucuuuu acggugcuga uggggagcag gugugccgau gcugcguauc cggucugacc 120
   cgcacgcacg cggagggacc gcaggcccuu ucaggaggcg ugaugagcca agcguggcga 180
   uagguaccaa uccaagccua augguugcgg ucgauaagcg ggcggcugcc auguaaucac 240
   caacggauaa uuugcgguuu ggcgugauga gcuugagguc aacgaggaag uucgcucaag 300
   guuucgcggu gc 312
<210> 22
   <211> 303
   <212> RNA
   <213> Artificial Sequence
<220>
   <223> fragment
<400> 22
   ggugaacagc ucguccgcac cgcgaaaccu ugagcgaacu uccucguuga ccucaagcuc 60
   ugaccaaacc gcaaauuauc cguuggugau uacauggcag ccgcccgcuu aucgaccgca 120
   accauuaggc uuggauuggu accuaucgcc acgcuuggcu cugaccuccu gaaagggccu 180
   gcggucccuc cgcgugcgug cgggucagac cggauacgca gcaucggcac accugcuccc 240
   caucagcacc guaaaagagc ucugaccaga guuuuggugu aauugguugg gucauguccg 300
   ugu 303
<210> 23
   <211> 12
   <212> RNA
   <213> Artificial Sequence
<220>
   <223> fragment
<400> 23
   ggcgucauga gc 12
<210> 24
   <211> 9
   <212> RNA
   <213> Artificial Sequence
<220>
   <223> fragment
<400> 24
   gcucugccc 9
<210> 25
   <211> 174
   <212> DNA
   <213> Artificial Sequence
<220>
   <223> affibody
<400> 25
   gtagacaaca aattcaacaa agaaatgcgg aacgcgtatt gggagatcgc gctgttacct 60
   aacttaaaca atcagcaaaa acgcgccttc atccgcagtt tatatgatga cccaagccaa 120
   agcgcgaatc tgctggcgga agccaagaaa ctgaatgatg cgcaggcacc gaaa 174
<210> 26
   <211> 58
   <212> PRT
   <213> Artificial Sequence
<220>
   <223> affibody
<400> 26
   Val Asp Asn Lys Phe Asn Lys Glu Met Arg Asn Ala Tyr Trp Glu Ile
   1               5                   10                  15
   Ala Leu Leu Pro Asn Leu Asn Asn Gln Gln Lys Arg Ala Phe Ile Arg
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 27
   <211> 45
   <212> DNA
   <213> Artificial Sequence
<220>
   <223> spacer
<400> 27
   agctcaagct caggctcaag tagttcaggt tcatcgagca gtggt 45
<210> 28
   <211> 12
   <212> PRT
   <213> Artificial Sequence
<220>
   <223> spacer
<400> 28
   Ser Ser Ser Gly Ser Ser Ser Gly Ser Ser Ser Gly
   1               5                   10
<210> 29
   <211> 63
   <212> RNA
   <213> Artificial Sequence
<220>
   <223> fragment
<400> 29
   ggcaucaagg ugaacuucaa gauccgcaaa ggccuguaau cggcgugaug agccaugcga        60
   gga                                                                      63
<210> 30
   <211> 63
   <212> RNA
   <213> Artificial Sequence
<220>
   <223> fragment
<400> 30
   ggcaucaagg ugaacuucaa gauccggaaa ugaaguccaa uggcgugaug agccucuacg 60
   gga 63
<210> 31
   <211> 63
   <212> RNA
   <213> Artificial Sequence
<220>
   <223> fragment
<400> 31
   ggcaucaagg ugaacuucaa gauccagagc augcccaucc gggcgugaug agcguagcaa 60
   ggc 63
<210> 32
   <211> 27
   <212> RNA
   <213> Artificial Sequence
<220>
   <223> fragment
<400> 32
   ggaucuugaa guucaccuug augccag 27
<210> 33
   <211> 59
   <212> RNA
   <213> Artificial Sequence
<220>
   <223> shRNA
<400> 33
   ggcaucaagg ugaacuucaa gauccagcau agggaucuug aaguucaccu ugaugccag 59